# EUROPÄISCHE PATENTSCHRIFT

(11) **EP 2 231 643 B1**
(45) Veröffentlichungstag und Bekanntmachung des Hinweises auf die Patenterteilung: **31.10.2012**
(21) Anmeldenummer: 08856714.4
(22) Anmeldetag: 05.12.2008
(51) Int. Cl.: C07D 401/14, A61K 31/496, A61P 3/00, A61P 7/00, A61P 9/00, A61P 25/00

(54) **Amidomethyl-substitutierte Oxindol-Derivate und ihre Verwendung zur Herstellung eines Medikaments zur Behandlung von Vasopressin-abhängigen Erkrankungen**
Amidomethyl-substituted oxindole derivatives and their use in the manufacture of a medicament for the treatment of vasopressin-dependent diseases
Dérivés oxindoliques substitués par amidométhyle et leur utilisation pour la préparation d'un medicament pour le traitement de maladies liées à la vasopressine

(30) Priorität: 07.12.2007 US 12241 P
(43) Veröffentlichungstag der Anmeldung: 29.09.2010
(73) Patentinhaber: Abbott GmbH & Co. KG, 65205 Wiesbaden (DE)
(72) Erfinder: OOST, Thorsten, 88400 Biberach An Der Riss (DE); NETZ, Astrid, 67061 Ludwigshafen (DE); GENESTE, Hervé, 67061 Ludwigshafen (DE); BRAJE, Wilfried, 67061 Ludwigshafen (DE); WERNET, Wolfgang, verstorben (DE); UNGER, Liliane, 67061 Ludwigshafen (DE); HORNBERGER, Wilfried, 67061 Ludwigshafen (DE); LUBISCH, Wilfried, 69118 Heidelberg (DE)
(74) Vertreter: Reitstötter - Kinzebach
(86) Internationale Anmeldenummer: PCT/EP2008/066931
(87) Internationale Veröffentlichungsnummer: WO 2009/071687

(56) Entgegenhaltungen:
- WO-A-2005/030755
- WO-A-2006/005609
- WO-A-2008/080973

## Beschreibung

Die vorliegende Erfindung betrifft neue substituierte Oxindol-Derivate, diese enthaltende pharmazeutische Mittel und ihre Verwendung zur Herstellung eines Medikaments zur Behandlung von Vasopressin-abhängigen Erkrankungen.

Vasopressin ist ein endogenes Hormon, das verschiedenste Wirkungen an Organen und Gewebe ausübt. Bei verschiedenen Krankheitszuständen, wie zum Beispiel Herzinsuffizienz und Bluthochdruck, vermutet man, dass das Vasopressin-System eine Rolle spielt. Derzeit sind drei Rezeptoren (V1a, V1b bzw. V3 und V2) bekannt, über die Vasopressin seine zahlreichen Wirkungen vermittelt. Daher werden Antagonisten dieser Rezeptoren als mögliche neue therapeutische Ansätze zur Behandlung von Krankheiten untersucht (M. Thibonnier, Exp.Opin. Invest. Drugs 1998, 7(5), 729-740).

Vorliegend werden neue substituierte Oxindole beschrieben, die in der 1-Position eine Phenylsulfonyl-Gruppe tragen. 1-Phenylsulfonyl-1,3-dihydro-2*H*-indol-2-one wurden bereits als Liganden der Vasopressin-Rezeptoren beschrieben. Auch die WO 93/15051, WO 95/18105, WO 98/25901, WO 01/55130, WO 01/55134, WO 01/164668 und WO 01/98295 beschreiben Derivate, die in der 1-Position des Oxindolgerüsts Arylsulfonlygruppen tragen. Diese Verbindungen unterscheiden sich von den erfindungsgemäßen Verbindungen wesentlich durch die Substituenten in der 3-Position.

So werden in der WO 93/15051 und WO 98/25901 1-Phenylsulfonyl-1,3-dihydro-2*H*-indol-2-one als Liganden der Vasopressinrezeptoren beschrieben, bei denen das Oxindol-Gerüst in der 3-Position durch zwei Alkylreste substituiert ist, die gemeinsam auch einen Cycloalkylrest (Spiroverknüpfung) bilden können. Als Alternative kann der Spiroring Heteroatome, wie Sauerstoff und Stickstoff (wahlweise mit Substituenten), enthalten.

Die WO 95/18105 beschreibt 1-Phenylsulfonyl-1,3-dihydro-2*H*-indol-2-one als Liganden der Vasopressinrezeptoren, die ein Stickstoffatom in der 3-Position besitzen. Zusätzlich sind in der 3-Position Reste gebunden, die unter gegebenenfalls substituierten Alkyl-, Cycloalkyl-, Phenyl- oder Benzylresten ausgewählt sind.

In der WO 03/008407 werden 1-Phenylsulfonyloxindole beschrieben, in denen in der 3-Position Pyridylpiperazine über eine Oxycarbonyl-Gruppe am Oxindol gebunden sind.

In der WO 2006/005609 werden 1-Phenylsulfonyl-oxindole beschrieben, in denen in 3-Stellung 4-(4-Propyl-piperazin-1-yl)-piperidin-Reste über eine 2-Oxo-ethyl-Gruppe am Oxindol gebunden sind, zum Beispiel 1-(2,4-Dimethoxy-phenylsulfonyl)-3-(2-ethoxyphenyl)-2-oxo-3-{2-oxo-2-[4-(4-propyl-piperazin-1-yl)-piperidin-1-yl]-ethyl}2,3-dihydro-1H-indol-5-carbonitril (Beispiel 194).

Neben der Bindungsaffinität zum Vasopressin V1 b Rezeptor können weitere Eigenschaften vorteilhaft für die Behandlung und/oder Prophylaxe von Vasopressin-abhängigen Erkrankungen sein, wie beispielsweise:
1.) eine Selektivität zum Vasopressin V1b Rezeptor im Vergleich zum Vasopressin V1a Rezeptor, d.h. der Quotient aus der Bindungsaffinität zum V1a Rezeptor (Ki(V1a) (bestimmt in der Einheit "Nanomolar (nM)") und der Bindungsaffinität zum V1 b Rezeptor (Ki(V1b)) (bestimmt in der Einheit "Nanomolar (nM)"). Je größer der Quotient Ki(V1a)/Ki(V1b) ist, desto größer ist die V1b-Selektivität;
2.) eine Selektivität zum Vasopressin V1b Rezeptor im Vergleich zum Vasopressin V2 Rezeptor, d.h. der Quotient aus der Bindungsaffinität zum V2 Rezeptor (Ki(V2) (bestimmt in der Einheit "Nanomolar (nM)") und der Bindungsaffinität zum V1 b Rezeptor (Ki(V1b)) (bestimmt in der Einheit "Nanomolar (nM)"). Je größer der Quotient Ki(V2)/Ki(V1 b) ist, desto größer ist die V1b-Selektivität;
3.) eine Selektivität zum Vasopressin V1 b Rezeptor im Vergleich zum Oxytozin OT Rezeptor, d.h. der Quotient aus der Bindungsaffinität zum OT Rezeptor (Ki(OT) (bestimmt in der Einheit "Nanomolar (nM)") und der Bindungsaffinität zum V1b Rezeptor (Ki(V1b)) (bestimmt in der Einheit "Nanomolar (nM)"). Je größer der Quotient Ki(OT)/Ki(V1 b) ist, desto größer ist die V1b-Selektivität.
4.) die metabolische Stabilität, beispielsweise bestimmt anhand der in-vitro ermittelten Halbwertszeiten in Lebermikrosomen von verschiedenen Spezies (z.B. Ratte oder Mensch);
5.) keine oder nur geringe Inhibierung von Cytochrom P450 (CYP) Enzymen: Cytochrom P450 (CYP) ist die Bezeichnung für eine Superfamilie von Hämproteinen mit enzymatischer Aktivität (Oxidase). Besondere Bedeutung besitzen sie auch für den Abbau (Metabolismus) von Fremdstoffen wie Pharmaka oder Xenobiotika in Säugetierorganismen. Die wichtigsten Vertreter der Typen und Untertypen von CYP im menschlichen Organismus sind: CYP 1A2, CYP 2C9, CYP 2D6 und CYP 3A4. Bei gleichzeitiger Anwendung von CYP 3A4-Hemmstoffen (z.B. Grapefruitsaft, Cimetidin, Erythromycin) und Arzneistoffen, die über dieses Enzymsystem abgebaut werden und somit um die gleiche Bindungsstelle am Enzym konkurrieren, kann deren Abbau verlangsamt und dadurch Wirkungen und Nebenwirkungen des verabreichten Arzneistoffs unerwünscht verstärkt werden;
6.) eine geeignete Wasserlöslichkeit (in mg/ml);
7.) eine geeignete Pharmakokinetik (zeitlicher Verlauf der Konzentration der erfindungsgemässen Verbindung im Plasma bzw. in Geweben, zum Beispiel Gehirn). Die Pharmakokinetik kann durch die folgenden Parameter beschrieben werden: Halbwertszeit, Verteilungsvolumen (in l·kg⁻¹), Plasma-Clearance (in l·h⁻¹·kg⁻¹), AUC ("area under the curve", Fläche unter der Konzentrations-Zeit-Kurve, (in ng·h·l⁻¹)), orale Bioverfügbarkeit (das Dosis-normalisierte Verhältnis von AUC nach oraler Gabe und AUC nach intravenöser Gabe), das sog. Brain-Plasma-Ratio (das Verhältnis von AUC im Hirngewebe und AUC im Plasma);
8.) keine oder nur geringe Blockade des hERG-Kanals: Verbindungen, die den hERG-Kanal blockieren, können eine Verlängerung des QT-Intervalls verursachen und dadurch zu ernsten Herz-Rhythmus-Störungen führen (zum Beispiel sogenannte "torsade de pointes"). Mittels eines in der Literatur beschriebenen Verdrängungsassays mit radioaktiv markiertem Dofetilid (G. J. Diaz et al., Journal of Pharmacological and Toxicological Methods, 50 (2004), 187-199) kann das Potential von Verbindungen, den hERG-Kanal zu blockieren, bestimmt werden. Je geringer der IC50 in diesem "dofetilide assay", desto wahrscheinlicher ist eine potente hERG-Blockade. Darüber hinaus kann die Blockade des hERG-Kanals durch elektrophysiologische Experimente an Zellen, die mit dem hERG-Kanal transfiziert wurden, durch sogenanntes "whole-cell patch clamping" gemessen werden (G. J. Diaz et al., Journal of Pharmacological and Toxicological Methods, 50 (2004), 187-199).

Aufgabe der vorliegenden Erfindung war es, Verbindungen zur Anwendung in einem Verfahren für die Behandlung oder Prophylaxe von verschiedenen Vasopressin-abhängigen Krankheiten zur Verfügung zu stellen. Die Verbindungen sollten eine hohe Aktivität und Selektivität aufweisen, vor allem eine hohe Affinität und Selektivität gegebenüber dem Vasopressin V1b Rezeptor. Zusätzlich sollte die erfindungsgemäße Substanz einen oder mehrere der vorstehend genannten Vorteile 1.) bis 8.) besitzen.

Die Aufgabe wird durch Verbindungen der Formel I gelost 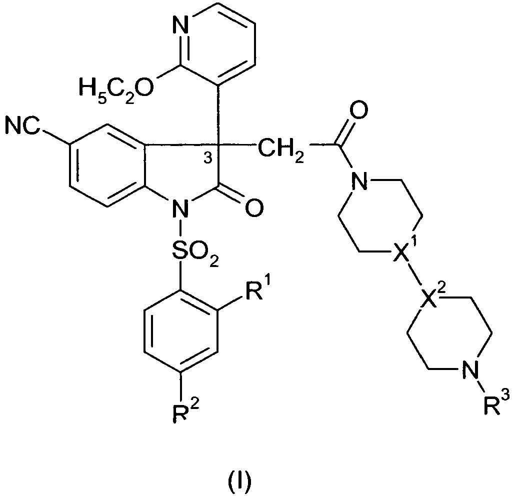 worin
- R¹: für Wasserstoff, Methoxy oder Ethoxy steht;
- R²: für Wasserstoff oder Methoxy steht;
- R³: für Wasserstoff, Methyl, Ethyl, n-Propyl oder Isopropyl steht;
- X¹ und X²: für N oder CH stehen, unter der Maßgabe, dass X¹ und X² nicht gleichzeitig für N stehen;
sowie durch deren pharmazeutisch verträglichen Salze.

Dementsprechend betrifft die vorliegende Erfindung Verbindungen der Formel I (im Folgenden auch "Verbindungen I") sowie die pharmazeutisch verträgliche Salze der Verbindungen I.

Die pharmazeutisch verträglichen Salze von Verbindungen der Formel I, sie auch als physiologisch verträgliche Salze bezeichnet werden, sind in der Regel durch Umsetzung der freien Base der erfindungsgemäßen Verbindungen I (d.h. der Verbindungen I gemäß Strukturformel I) mit geeigneten Säuren erhältlich. Geeignete Säuren sind beispielsweise aufgeführt in "Fortschritte der Arzneimittelforschung", 1966, Birkhäuser Verlag, Bd.1 0, S.224-285. Darunter fallen beispielsweise Salzsäure, Zitronensäure, Weinsäure, Milchsäure, Phosphorsäure, Methansulfonsäure, Essigsäure, Ameisensäure, Maleinsäure und Fumarsäure.

C₁-C₄-Alkyl steht im Rahmen der vorliegenden Erfindung für einen linearen oder verzweigten Alkylrest mit 1 bis 4 Kohlenstoffatomen, wie Methyl, Ethyl, n-Propyl, Isopropyl, n-Butyl, sek-Butyl, Isobutyl oder tert-Butyl.

C₁-C₃-Alkoxy steht im Rahmen der vorliegenden Erfindung für einen über ein Sauerstoffatom gebundenen linearen oder verzweigten Alkylrest mit 1 bis 3 Kohlenstoffatomen. Beispiele sind Methoxy, Ethoxy, n-Propoxy und Isopropoxy.

Die erfindungsgemäßen Verbindungen der Formel I, ihre pharmakologisch verträglichen Salze können auch in Form von Solvaten oder Hydraten vorliegen. Unter Solvaten versteht man im Rahmen der vorliegenden Erfindung kristalline Formen der Verbindungen I bzw. ihrer pharmazeutisch verträglichen Salze, die im Kristallgitter Lösungsmittelmoleküle eingebaut enthalten. Vorzugsweise sind die Lösungsmittelmoleküle in stöchiometrischen Verhältnissen eingebaut. Hydrate sind eine Spezialform der Solvate; das Lösungsmittel ist hier Wasser.

Die nachstehend gemachten Angaben zu geeigneten und bevorzugten Merkmalen der Erfindung, insbesondere zu den Resten R¹, R², R³, X¹ und X² der Verbindung I, aber auch zu den Merkmalen des erfindungsgemäßen Verfahrens und der erfindungsgemäßen Verwendung gelten sowohl für sich allein genommen als auch vorzugsweise in jeder möglichen Kombination miteinander.

Die Verbindungen I werden bevorzugt in Form der freien Base (d.h. gemäß der Strukturformel I) oder in Form ihrer Säureadditionssalze bereitgestellt.

In einer bevorzugten Ausführungsform steht R¹ für Wasserstoff oder Methoxy.

In einer weiteren bevorzugten Ausführungsform steht R³ für Wasserstoff, Methyl oder Ethyl.

In einer weiteren bevorzugten Ausführungsform steht eine der Variablen X¹, X² für N und die andere für CH.

In einer besonders bevorzugten Ausführungsform steht dabei X¹ für N und X² steht für CH.

In einer alternativ besonders bevorzugten Ausführungsform steht X¹ für CH und X² steht für N.

Ein bevorzugter Gegenstand der Erfindung sind Verbindungen der Formel I, worin
- R¹: Wasserstoff oder Methoxy ist;
- R²: Wasserstoff oder Methoxy ist;
- R³: Wasserstoff, Methyl, Ethyl, n-Propyl oder Isopropyl; vorzugsweise Wasserstoff, Methyl oderEthyl ist;
- X¹: N oder CH ist;
- X²: N oder CH ist;
wobei X¹ und X² nicht gleichzeitig für N stehen; sowie die pharmazeutisch verträglichen Salze davon. In einer besonderen Ausführungsform stehen dabei die Variablen X¹ und X² nicht gleichzeitig für CH, d.h. vorzugsweise steht eine der Variablen X¹ oder X² für N und die andere steht für CH.

Ein besonders bevorzugter Gegenstand der Erfindung sind Verbindungen der Formel I, worin
- R¹: Wasserstoff oder Methoxy ist;
- R²: Wasserstoff oder Methoxy ist;
- R³: Wasserstoff, Methyl oder Ethyl ist;
- X¹: N ist;
- X²: CH ist;
sowie die pharmazeutisch verträglichen Salze davon.

Bevorzugt ist hierunter die Verbindung I, worin
- R¹: für Methoxy steht;
- R²: für Methoxy steht;
- R³: für Methyl steht;
- X¹: für N steht; und
- X²: für CH steht;
und die Verbindung I, worin
- R¹: für Methoxy steht;
- R²: für Methoxy steht;
- R³: für Ethyl steht;
- X¹: für N steht; und
- X²: für CH steht;
wobei die erste Verbindung stärker bevorzugt ist.

Ein alternativ besonders bevorzugter Gegenstand der Erfindung sind Verbindungen der Formel I, worin
- R¹: Wasserstoff oder Methoxy ist;
- R²: Wasserstoff oder Methoxy ist;
- R³: Wasserstoff, Methyl oder Ethyl ist;
- X¹: CH ist;
- X²: N ist;
sowie die pharmazeutisch verträglichen Salze davon.

Bevorzugt ist hierunter die Verbindung I, worin
- R¹: für Methoxy steht;
- R²: für Methoxy steht;
- R³: für Methyl steht;
- X¹: für CH steht; und
- X²: für N steht;
und die Verbindung I, worin
- R¹: für Methoxy steht;
- R²: für Methoxy steht;
- R³: für Ethyl steht;
- X¹: für CH steht; und
- X²: für N steht;
wobei die erste Verbindung stärker bevorzugt ist.

Ein weiterer besonders bevorzugter Gegenstand der Erfindung sind Verbindungen der Formel I, worin
- R¹: Wasserstoff oder Methoxy ist;
- R²: Wasserstoff oder Methoxy ist;
- R³: Wasserstoff, Methyl oder Ethyl ist;
- X¹: CH ist;
- X²: CH ist;
sowie die pharmazeutisch verträglichen Salze davon.

Bevorzugt ist hierunter die Verbindung I, worin
- R¹: für Methoxy steht;
- R²: für Methoxy steht;
- R³: für Methyl steht;
- X¹: für CH steht; und
- X²: für CH steht;
und die Verbindung I, worin
- R¹: für Methoxy steht;
- R²: für Methoxy steht;
- R³: für Ethyl steht;
- X¹: für CH steht; und
- X²: für CH steht;
wobei die erste Verbindung stärker bevorzugt ist.

Beispiele für bevorzugte Ausführungsform der vorliegenden Erfindung sind Verbindungen der Formel I sowie die pharmazeutisch verträglichen Salze davon, worin die Reste X¹, X², R¹, R² und R³ jeweils die in der folgenden Tabelle 1 pro Zeile genannten Bedeutungen annehmen.

**Tabelle 1:**

| **Verbindung Nr.** | **X¹** | **X²** | **R¹** | **R²** | **R³** |
|---|---|---|---|---|---|
| I-1 | N | CH | Methoxy | Methoxy | Methyl |
| I-2 | N | CH | Methoxy | H | Methyl |
| I-3 | N | CH | Ethoxy | H | Methyl |
| I-4 | N | CH | H | H | Methyl |
| I-5 | N | CH | H | Methoxy | Methyl |
| I-6 | N | CH | Ethoxy | Methoxy | Methyl |
| I-7 | N | CH | Methoxy | Methoxy | Ethyl |
| I-8 | N | CH | Methoxy | H | Ethyl |
| I-9 | N | CH | Ethoxy | H | Ethyl |
| I-10 | N | CH | H | H | Ethyl |
| I-11 | N | CH | H | Methoxy | Ethyl |
| I-12 | N | CH | Ethoxy | Methoxy | Ethyl |
| I-13 | N | CH | Methoxy | Methoxy | n-Propyl |
| I-14 | N | CH | Methoxy | H | n-Propyl |
| I-15 | N | CH | Ethoxy | H | n-Propyl |
| I-16 | N | CH | H | H | n-Propyl |
| I-17 | N | CH | H | Methoxy | n-Propyl |
| I-18 | N | CH | Ethoxy | Methoxy | n-Propyl |
| I-19 | N | CH | Methoxy | Methoxy | Isopropyl |
| I-20 | N | CH | Methoxy | H | Isopropyl |
| I-21 | N | CH | Ethoxy | H | Isopropyl |
| I-22 | N | CH | H | H | Isopropyl |
| I-23 | N | CH | H | Methoxy | Isopropyl |
| I-24 | N | CH | Ethoxy | Methoxy | Isopropyl |
| I-25 | N | CH | Methoxy | Methoxy | H |
| I-26 | N | CH | Methoxy | H | H |
| I-27 | N | CH | Ethoxy | H | H |
| I-28 | N | CH | H | H | H |
| I-29 | N | CH | H | Methoxy | H |
| I-30 | N | CH | Ethoxy | Methoxy | H |
| I-31 | CH | N | Methoxy | Methoxy | Methyl |
| I-32 | CH | N | Methoxy | H | Methyl |
| I-33 | CH | N | Ethoxy | H | Methyl |
| I-34 | CH | N | H | H | Methyl |
| I-35 | CH | N | H | Methoxy | Methyl |
| I-36 | CH | N | Ethoxy | Methoxy | Methyl |
| I-37 | CH | N | Methoxy | Methoxy | Ethyl |
| I-38 | CH | N | Methoxy | H | Ethyl |
| I-39 | CH | N | Ethoxy | H | Ethyl |
| I-40 | CH | N | H | H | Ethyl |
| I-41 | CH | N | H | Methoxy | Ethyl |
| I-42 | CH | N | Ethoxy | Methoxy | Ethyl |
| I-43 | CH | N | Methoxy | Methoxy | n-Propyl |
| I-44 | CH | N | Methoxy | H | n-Propyl |
| I-45 | CH | N | Ethoxy | H | n-Propyl |
| I-46 | CH | N | H | H | n-Propyl |
| I-47 | CH | N | H | Methoxy | n-Propyl |
| I-48 | CH | N | Ethoxy | Methoxy | n-Propyl |
| I-49 | CH | N | Methoxy | Methoxy | Isopropyl |
| I-50 | CH | N | Methoxy | H | Isopropyl |
| I-51 | CH | N | Ethoxy | H | Isopropyl |
| I-52 | CH | N | H | H | Isopropyl |
| I-53 | CH | N | H | Methoxy | Isopropyl |
| I-54 | CH | N | Ethoxy | Methoxy | Isopropyl |
| I-55 | CH | N | Methoxy | Methoxy | H |
| I-56 | CH | N | Methoxy | H | H |
| I-57 | CH | N | Ethoxy | H | H |
| I-58 | CH | N | H | H | H |
| I-59 | CH | N | H | Methoxy | H |
| I-60 | CH | N | Ethoxy | Methoxy | H |
| I-61 | CH | CH | Methoxy | Methoxy | Methyl |
| I-62 | CH | CH | Methoxy | H | Methyl |
| I-63 | CH | CH | Ethoxy | H | Methyl |
| I-64 | CH | CH | H | H | Methyl |
| I-65 | CH | CH | H | Methoxy | Methyl |
| I-66 | CH | CH | Ethoxy | Methoxy | Methyl |
| I-67 | CH | CH | Methoxy | Methoxy | Ethyl |
| I-68 | CH | CH | Methoxy | H | Ethyl |
| I-69 | CH | CH | Ethoxy | H | Ethyl |
| I-70 | CH | CH | H | H | Ethyl |
| I-71 | CH | CH | H | Methoxy | Ethyl |
| I-72 | CH | CH | Ethoxy | Methoxy | Ethyl |
| I-73 | CH | CH | Methoxy | Methoxy | n-Propyl |
| I-74 | CH | CH | Methoxy | H | n-Propyl |
| I-75 | CH | CH | Ethoxy | H | n-Propyl |
| I-76 | CH | CH | H | H | n-Propyl |
| I-77 | CH | CH | H | Methoxy | n-Propyl |
| I-78 | CH | CH. | Ethoxy | Methoxy | n-Propyl |
| I-79 | CH | CH | Methoxy | Methoxy | Isopropyl |
| I-80 | CH | CH | Methoxy | H | Isopropyl |
| I-81 | CH | CH | Ethoxy | H | Isopropyl |
| I-82 | CH | CH | H | H | Isopropyl |
| I-83 | CH | CH | H | Methoxy | Isopropyl |
| I-84 | CH | CH | Ethoxy | Methoxy | Isopropyl |
| I-85 | CH | CH | Methoxy | Methoxy | H |
| I-86 | CH | CH | Methoxy | H | H |
| I-87 | CH | CH | Ethoxy | H | H |
| I-88 | CH | CH | H | H | H |
| I-89 | CH | CH | H | Methoxy | H |
| I-90 | CH | CH | Ethoxy | Methoxy | H |

Die erfindungsgemäßen Verbindungen I weisen in der 3-Position des 2-Oxindolrings ein Chiralitätszentrum auf. Die erfindungsgemäßen Verbindungen können daher als ein 1:1-Gemisch von Enantiomeren (Racemat) oder als ein nicht-racemisches Gemisch von Enantiomeren, in dem eines der beiden Enantiomere, entweder das die Schwingungsebene von linear polarisiertem Licht nach links drehende (d.h. minusdrehende) Enantiomer (im Folgenden (-)-Enantiomer) oder das die Schwingungsebene von linear polarisiertem Licht nach rechts drehende (d.h. plusdrehende) Enantiomer (im Folgenden (+)-Enantiomer), angereichert ist, oder als im Wesentlichen enantiomerenreine Verbindungen, also als im Wesentlichen enantiomerenreines (-)-Enantiomer oder (+)-Enantiomer, vorliegen. Da bei den erfindungsgemäßen Verbindungen ein einziges Asymmetriezentrum und keine Chiralitätsachse/-ebene existiert, kann man ein nicht-racemisches Gemisch auch als ein Gemisch von Enantiomeren definieren, in welchem entweder das R- oder das S-Enantiomer überwiegt. Im Wesentlichen enantiomerenreine Verbindungen können dementsprechend auch als im Wesentlichen enantiomerenreines R-Enantiomer bzw. im Wesentlichen enantiomerenreines S-Enantiomer definiert werden.

Unter "im Wesentlichen enantiomerenreinen Verbindungen" versteht man im Rahmen der vorliegenden Erfindung solche Verbindungen, die einen Enantiomerenüberschuss (enantiomeric excess, ee; % ee = (R-S)/(R+S) x 100 bzw. (S-R)/(S+R) x 100) von wenigstens 80 % ee, vorzugsweise wenigsten 85 % ee, stärker bevorzugt wenigstens 90 % ee, noch stärker bevorzugt wenigstens 95 % ee und insbesondere wenigstens 98 % ee aufweisen.

In einer Ausführungsform der Erfindung liegen die erfindungsgemäßen Verbindungen als im Wesentlichen enantiomerenreine Verbindungen vor. Besonders bevorzugt sind Verbindungen, die einen Enantiomerenüberschuss von wenigstens 85 % ee, stärker bevorzugt von wenigstens 90 % ee, noch stärker bevorzugt von wenigstens 95 % ee und insbesondere von wenigstens 98 % ee aufweisen.

Gegenstand der Erfindung sind somit sowohl die reinen Enantiomere als auch deren Gemische, z.B. Gemische, in denen ein Enantiomer in angereichter Form vorliegt, aber auch die Racemate. Gegenstand der Erfindung sind auch die pharmazeutisch verträglichen Salze der reinen Enantiomere von Verbindungen I sowie die Enantiomerengemische in Form der pharmazeutisch verträglichen Salze von Verbindungen I.

Bevorzugte Ausführungsformen der Erfindung sind Verbindungen der Formel I, wie vorstehend ausgeführt, die dadurch gekennzeichnet sind, dass sie in optisch aktiver Form vorliegen und es sich jeweils um das die Schwingungsebene von polarisiertem Licht nach links drehende (d.h. das minusdrehende) Enantiomer der betreffenden Verbindung der Formel I in der Form der freien Base handelt, oder um ein pharmazeutisch verträgliches Salz davon. Die links- bzw. minusdrehenden Enantiomere der Verbindungen I werden im Folgenden auch als (-)-Enantiomere bezeichnet.

Besonders bevorzugt sind Verbindungen der allgemeinen Formel I und deren pharmazeutisch verträglichen Salze, wie vorstehend ausgeführt, worin das entsprechende (-)-Enantiomer in einer optischen Reinheit (enantiomeric excess, ee) von größer 50 % ee, besonders bevorzugt von wenigstens 80 % ee, stärker bevorzugt von wenigstens 90 % ee, noch stärker bevorzugt von wenigstens 95 % ee und insbesondere von wenigstens 98 % ee vorliegt.

Ebenfalls bevorzugte Ausführungsformen der Erfindung sind Verbindungen der allgemeinen Formel I, wie vorstehend ausgeführt, die dadurch gekennzeichnet sind, dass sie in optisch inaktiver Form, d.h. in Form des Racemats, vorliegen, oder in Form eines pharmazeutisch verträglichen Salzes des Racemats.

Die im Rahmen der vorliegenden Erfindung gemachten Angaben zur Drehrichtung des polarisierten Lichts beziehen sich vorzugsweise auf Vorzeichen [(+) oder (-)] wie sie in Chloroform als Lösungsmittel oder in Chloroform-haltigen Lösungsmittelgemischen, insbesondere in Chloroform, ermittelt werden.

Beispiele für besonders bevorzugte Verbindungen I sind die in nachfolgender Tabelle 2 aufgeführten Verbindungen mit den Verbindungsnummern I-1, I-1A, I-1B, I-2, I-2A, I-2B, I-4, I-4A, I-4B, I-5, I-5A, I-5B, I-7, I-7A, I-7B, I-8, I-8A, I-8B, I-10, I-10A, I-10B, I-11, I-11A, I-11 B, I-31, I-31A, I-31B, I-32, I-32A, I-32B, 1-34, I-34A, I-34B, I-35, I-35A, I-35B, I-37, I-37A, I-37B, I-38, I-38A, I-38B, I-40, I-40A, I-40B, I-41, I-41A, I-41B, I-61, I-61A und I-61B sowie die pharmazeutisch verträglichen Salze davon. Dabei entsprechen die Beispiele, die nur durch Ziffern bezeichnet sind, dem Racemat der jeweiligen Verbindungen, die Beispiele mit einem angehängten Buchstaben "A" (1A, 2A, ...) dem rechtsdrehenden (+)-Enantiomer und die Beispiele mit einem angehängten Buchstaben "B" (1 B, 2B, ...) dem linksdrehenden (-)-Enantiomer der jeweiligen Verbindung 1, 2, ....

**Tabelle 2**

| **Verbindung Nr.** | **X¹** | **X²** | **R¹** | **R²** | **R³** | **IUPAC Name** |
|---|---|---|---|---|---|---|
| I-1 | N | CH | Methoxy | Methoxy | Methyl | *(*±*)*-1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indole-5-carbonitril |
| I-1A | N | CH | Methoxy | Methoxy | Methyl | *(*+*)*-1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril |
| I-1B | N | CH | Methoxy | Methoxy | Methyl | *(*-*)*-1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril |
| I-2 | N | CH | Methoxy | H | Methyl | *(*±*)*-3-(2-Ethoxy-pyridin-3-yl)-1-(2-methoxy-phenylsulfonyl)-3-{2-[4-(1-methyl-piperidin-4-yl)-piperazin-1-yl]-2-oxo-ethyl}-2-oxo-2,3-dihydro-1H-indol-5-carbonitril |
| I-2A | N | CH | Methoxy | H | Methyl | *(*+*)*-3-(2-Ethoxy-pyridin-3-yl)-1-(2-methoxy-phenylsulfonyl)-3-{2-[4-(1-methyl-piperidin-4-yl)-piperazin-1-yl]-2-oxo-ethyl}-2-oxo-2,3-dihydro-1H-indol-5-carbonitril |
| I-2B | N | CH | Methoxy | H | Methyl | *(*-*)*-3-(2-Ethoxy-pyridin-3-yl)-1-(2-methoxy-phenylsulfonyl)-3-{2-[4-(1-methyl-piperidin-4-yl)-piperazin-1-yl]-2-oxo-ethyl}-2-oxo-2,3-dihydro-1H-indol-5-carbonitril |
| I-4 | N | CH | H | H | Methyl | *(*±*)*-1-Phenylsulfonyl-3-(2-ethoxy-pyridin-3-yl)-3-{2-[4-(1-methyl-piperidin-4- |
| | | | | | | yl)-piperazin-1-yl]-2-oxo-ethyl}-2-oxo-2,3-dihydro-1H-indol-5-carbonitril |
| I-4A | N | CH | H | H | Methyl | *(*+*)*-1-Phenylsulfonyl-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril |
| I-4B | N | CH | H | H | Methyl | *(*-*)*-1-Phenylsulfonyl-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril |
| I-5 | N | CH | H | Methoxy | Methyl | *(*±*)*-3-(2-Ethoxy-pyridin-3-yl)-1-(4-methoxy-phenylsulfonyl)-3-{2-[4-(1 1-methyl-piperidin-4-yl)-piperazin-1-yl]-2-oxo-ethyl}-2-oxo-2,3-dihydro-1H-indol-5-carbonitril |
| I-5A | N | CH | H | Methoxy | Methyl | *(*+*)*-3-(2-Ethoxy-pyridin-3-yl)-1-(4-methoxy-phenylsulfonyl)-3-{2-[4-(1-methyl-piperidin-4-yl)-piperazin-1-yl]-2-oxo-ethyl}-2-oxo-2,3-dihydro-1H-indol-5-carbonitril |
| I-5B | N | CH | H | Methoxy | Methyl | *(*-*)*-3-(2-Ethoxy-pyridin-3-yl)-1-(4-methoxy-phenylsulfonyl)-3-{2-[4-(1-methyl-piperidin-4-yl)-piperazin-1-yl]-2-oxo-ethyl}-2-oxo-2,3-dihydro-1H-indol-5-carbonitril |
| I-7 | N | CH | Methoxy | Methoxy | Ethyl | *(*±*)*-1-(2,4-Dimethoxy-phenylsulfonyl)-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril |
| I-7A | N | CH | Methoxy | Methoxy | Ethyl | *(*+*)*-1-(2,4-Dimethoxy-phenylsulfonyl)-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril |
| I-7B | N | CH | Methoxy | Methoxy | Ethyl | *(*-*)*-1-(2,4-Dimethoxy-phenylsulfonyl)-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril |
| I-8 | N | CH | Methoxy | H | Ethyl | *(*±*)*-3-(2-Ethoxy-pyridin-3-yl)-3-{2-[4-(1-ethyl-piperidin-4-yl)-piperazin-1-yl]-2-oxo-ethyl}-1-(2-methoxy-phenylsulfonyl)-2-oxo-2,3-dihydro-1H-indol-5-carbonitril |
| I-8A | N | CH | Methoxy | H | Ethyl | *(*+*)*-3-(2-Ethoxy-pyridin-3-yl)-3-{2-[4-(1-ethyl-piperidin-4-yl)-piperazi n-1-yl]-2-oxo-ethyl}-1-(2-methoxy-phenylsulfonyl)-2-oxo-2,3-dihydro-1H-indol-5-carbonitril |
| I-8B | N | CH | Methoxy | H | Ethyl | *(*-*)*-3-(2-Ethoxy-pyridin-3-yl)-3-{2-[4-(1-ethyl-piperidin-4-yl)-piperazin-1-yl]-2-oxo-ethyl}-1-(2-methoxy-phenylsulfonyl)-2-oxo-2,3-dihydro-1H-indol-5-carbonitril |
| I-10 | N | CH | H | H | Ethyl | *(*±*)-*1-Phenylsulfonyl-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril |
| I-10A | N | CH | H | H | Ethyl | *(*+*)*-1-Phenylsulfonyl-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril |
| I-10B | N | CH | H | H | Ethyl | *(*-*)*-1-Phenylsulfonyl-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril |
| I-11 | N | CH | H | Methoxy | Ethyl | *(*±*)*-3-(2-Ethoxy-pyridin-3-yl)-3-{2-[4-(1-ethyl-piperidin-4-yl)-piperazin-1 -yl]-2-oxo-ethyl}-1-(4-methoxy-phenylsulfonyl)-2-oxo-2,3-dihydro-1H-indol-5- |
| | | | | | | carbonitril |
| I-11A | N | CH | H | Methoxy | Ethyl | *(*+*)*-3-(2-Ethoxy-pyridin-3-yl)-3-{2-[4-(1-ethyl-piperidin-4-yl)-piperazin-1-yl]-2-oxo-ethyl}-1-(4-methoxy-phenylsulfonyl)-2-oxo-2,3-dihydro-1H-indol-5-carbonitril |
| I-11B | N | CH | H | Methoxy | Ethyl | *(*-*)*-3-(2-Ethoxy-pyridin-3-yl)-3-{2-[4-(1-ethyl-piperidin-4-yl)-piperazin-1-yl]-2-oxo-ethyl}-1-(4-methoxy-phenylsulfonyl)-2-oxo-2,3-dihydro-1H-indol-5-carbonitril |
| I-31 | CH | N | Methoxy | Methoxy | Methyl | *(*±*)*-1-(2,4-Dimethoxy-phenylsulfonyl)-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril |
| I-31A | CH | N | Methoxy | Methoxy | Methyl | *(*+*)*-1-(2,4-Dimethoxy-phenylsulfonyl)-3-{2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril |
| I-31B | CH | N | Methoxy | Methoxy | Methyl | *(*-*)-*1-(2,4-Dimethoxy-phenylsulfonyl)-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril |
| I-32 | CH | N | Methoxy | H | Methyl | *(*±*)*-3-(2-Ethoxy-pyridin-3-yl)-1-(2-methoxy-phenylsulfonyl)-3-{2-[4-(4-methyl-piperazin-1-yl)-piperidin-1-yl]-2-oxo-ethyl}-2-oxo-2,3-dihydro-1H-indol-5-carbonitril |
| I-32A | CH | N | Methoxy | H | Methyl | *(*+*)-*3-(2-Ethoxy-pyridin-3-yl)-1-(2-methoxy-phenylsulfonyl)-3-(2-[4-(4-methyl- |
| | | | | | | piperazin-1-yl)-piperidin-1-yl}-2-oxo-ethyl}-2-oxo-2,3-dihydro-1H-indol-5-carbonitril |
| I-32B | CH | N | Methoxy | H | Methyl | *(*-*)*-3-(2-Ethoxy-pyridin-3-yl)-1-(2-methoxy-phenylsulfonyl)-3-{2-[4-(4-methyl-piperazin-1-yl)-piperidin-1-yl]-2-oxo-ethyl}-2-oxo-2,3-dihydro-1H-indol-5-carbonitril |
| I-34 | CH | N | H | H | Methyl | *(*±*)*-1-Phenylsulfonyl-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril |
| I-34A | CH | N | H | H | Methyl | *(*+)*-*1-Phenylsulfonyl-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril |
| I-34B | CH | N | H | H | Methyl | *(*-*)*-1-Phenylsulfonyl-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril |
| I-35 | CH | N | H | Methoxy | Methyl | *(*±*)-*3-(2-Ethoxy-pyridin-3-yl)-1-(4-methoxy-phenylsulfonyl)-3-{2-[4-(4-methyl-piperazin-1-yl)-piperidin-1-yl]-2-oxo-ethyl}-2-oxo-2,3-dihydro-1H-indol-5-carbonitril |
| I-35A | CH | N | H | Methoxy | Methyl | *(*+*)*-3-(2-Ethoxy-pyridin-3-yl)-1-(4-methoxy-phenylsulfonyl)-3-{2-[4-(4-methyl-piperazin-1-yl)-piperidin-1-yl]-2-oxo-ethyl}-2-oxo-2,3-dihydro-1H-indol-5-carbonitril |
| I-35B | CH | N | H | Methoxy | Methyl | *(*-*)-*3-(2-Ethoxy-pyridin-3-yl)-1-(4-methoxy-phenylsulfonyl)-3-{2-[4-(4-methyl-piperazin-1-yl)-piperidin-1-yl]-2-oxo-ethyl}-2-oxo-2,3-dihydro-1H-indol-5-carbonitril |
| I-37 | CH | N | Methoxy | Methoxy | Ethyl | *(*±*)*-1-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril |
| I-37A | CH | N | Methoxy | Methoxy | Ethyl | *(*+*)*-1-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril |
| I-37B | CH | N | Methoxy | Methoxy | Ethyl | *(*-*)-*1-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril |
| I-38 | CH | N | Methoxy | H | Ethyl | *(*±*)*-3-(2-Ethoxy-pyridin-3-yl)-3-{2-[4-(4-ethyl-piperazin-1-yl)-piperidin-1 -yl]-2-oxo-ethyl}-1-(2-methoxy-phenylsulfonyl)-2-oxo-2,3-dihydro-1H-indol-5-carbonitril |
| I-38A | CH | N | Methoxy | H | Ethyl | *(*+*)*-3-(2-Ethoxy-pyridin-3-yl)-3-{2-[4-(4-ethyl-piperazin-1-yl)-piperid in-1-yl]-2-oxo-ethyl}-1-(2-methoxy-phenylsulfonyl)-2-oxo-2,3-dihydro-1H-indol-5-carbonitril |
| I-38B | CH | N | Methoxy | H | Ethyl | *(*-*)*-3-(2-Ethoxy-pyridin-3-yl)-3-{2-[4-(4-ethyl-piperazin-1-yl)-piperidin-1-yl]-2-oxo-ethyl}-1-(2-methoxy-phenylsulfonyl)-2-oxo-2,3-dihydro-1H-indol-5-carbonitril |
| I-40 | CH | N | H | H | Ethyl | *(*±*)-*1-Phenylsulfonyl-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril |
| I-40A | CH | N | H | H | Ethyl | *(*+*)*-1-Phenylsulfonyl-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril |
| I-408 | CH | N | H | H | Ethyl | *(*-*)-*1-Phenylsulfonyl-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril |
| I-41 | CH | N | H | Methoxy | Ethyl | *(*±*)*-3-(2-Ethoxy-pyridin-3-yl)-3-{2-[4-(4-ethyl-piperazin-1-yl)-piperid in-1-yl]-2-oxo-ethyl}-1-(4-methoxy-phenylsulfonyl)-2-oxo-2,3-dihydro-1H-indol-5-carbonitril |
| I-41A | CH | N | H | Methoxy | Ethyl | *(*+*)-*3-(2-Ethoxy-pyridin-3-yl)-3-{2-[4-(4-ethyl-piperazin-1-yl)-piperidin-1-yl]-2-oxo-ethyl}-1-(4-methoxy-phenylsulfonyl)-2-oxo-2,3-dihydro-1H-indol-5-carbonitril |
| I-41B | CH | N | H | Methoxy | Ethyl | *(*-*)*-3-(2-Ethoxy-pyridin-3-yl)-3-{2-[4-(4-ethyl-piperazin-1-yl)-piperidin-1-yl]-2-oxo-ethyl}-1-(4-methoxy-phenylsulfonyl)-2-oxo-2,3-dihydro-1H-indol-5-carbonitril |
| I-61 | CH | CH | Methoxy | Methoxy | Methyl | *(*±*)*-1-(2,4-Dimethoxyphenylsulfonyl)-3-(2-ethoxypyridin-3-yl)-3-[2-(1'-methyl-[4,4']bipiperidinyl-1-yl)-2-oxo-ethyl]-2-oxo-2,3-dihydro-1H-indol-5-carbonitril |
| I-61A | CH | CH | Methoxy | Methoxy | Methyl | *(*+*)*-1-(2,4-Dimethoxyphenylsulfonyl)-3-(2-ethoxypyridin-3-yl)-3-[2-(1'-methyl-[4*,*4']bipiperidinyl-1-yl)-2-oxo-ethyl]-2-oxo-2,3-dihydro-1H-indol-5-carbonitril |
| I-61B | CH | CH | Methoxy | Methoxy | Methyl | *(*-*)*-1-(2,4-Dimethoxyphenylsulfonyl)-3-(2-ethoxypyridin-3-yl)-3-[2-(1'-methyl-[4*,*4']bipiperidinyl-1-yl)-2-oxo-ethyl]-2-oxo-2,3-dihydro-1H-indol-5-carbonitril |

Hierunter besonders bevorzugt sind die Racemate (d.h. Verbindungen 1, 2, ...) und ihre physiologisch verträglichen Salze. Besonders bevorzugt sind auch die (-)-Enantiomeren (d.h. Verbindungen 1 B, 2B, ....) und ihre physiologisch verträglichen Salze. Insbesondere werden die zuvor genannten Verbindungen in Form ihrer freien Base oder in Form ihrer Säureadditionssalze bereitgestellt.

Im Folgenden werden beispielhafte Synthesewege zur Herstellung der erfindungsgemä-βen Oxindol-Derivate beschrieben.

Die Herstellung der erfindungsgemäßen Oxindole kann auf verschiedenen Wegen erfolgen und ist im Syntheseschema skizziert. In diesem Syntheseschema besitzen die Variablen die gleichen Bedeutungen wie in der Formel I.

Die 3-Hydroxy-1,3-dihydroindol-2-one IV können durch Addition von metallierten Heterocyclen III an die 3-Ketogruppe der Isatine II erhalten werden. Die metallierten Heterocyclen, wie beispielsweise die entsprechenden Grignard- (Mg) oder Organyllithium-Verbindung, können in üblicher Weise aus Halogen- oder Kohlenwasserstoffverbindungen erhalten werden. Beispielhafte Vorschriften sind in Houben-Weyl, Methoden der Organischen Chemie, Bd. 13, 1-2, Kap. Mg- bzw. Li-Verbindungen, sowie in der WO 2005/030755 und WO 2006/005609 enthalten. Die Isatine II sind entweder kommerziell erhältlich oder können in Analogie zu in der Literatur beschriebenen Methoden hergestellt werden (Advances in Heterocyclic Chemistry, A.R. Katritzky and A.J. Boulton, Academic Press, New York, 1975, 18, 2-58; J. Brazil. Chem. Soc. 12, 273-324, 2001).

Die 3-Hydroxyoxindole IV, die im 6-Ring-Aromaten beispielsweise in der 5-Position als Rest R^{a} ein lod enthalten, können mit KCN oder Zn(CN)₂ unter Pd(0)-Katalyse in Lösungsmitteln wie Dimethylformamid oder Tetrahydrofuran, gegebenenfalls auch unter Zusatz von Basen wie K₂CO₃ oder anderen Carbonaten oder Aminen, bei höherer Temperatur in das analoge cyanhaltige 3-Hydroxyoxindol IV überführt werden. Als Pd(0)Salze kann man zum Beispiel Übergangsmetallkomplexe nehmen, die in situ aus PdCl₂ oder PdOAc₂ durch Zugabe von Phosphinen wie Tris(ortho-tolyl)phosphin hergestellt werden. Ebenso können kommerzielle Palladium-Komplexe wie beispielsweise der Katalysator Tetrakis(triphenylphosphin)-palladium(0) und / oder Zusätze von Phosphin-Liganden eingesetzt werden. Beispiele zur Cyanierung von 5-lod-substituierten Oxindolen finden sich in WO 2005/030755 und WO 2006/005609.

Die 3-Hydroxy-oxindole IV können in die Verbindungen V überführt werden, welche eine Fluchtgruppe LG in 3-Stellung tragen, wobei die Fluchtgruppe LG eine übliche Abgangsgruppe sein kann, wie zum Beispiel Halogenid, Mesylat oder Tosylat. Das Zwischenprodukt V, mit beispielsweise LG = Chlor, kann durch Behandlung des Alkohols IV mit Thionylchlorid in Gegenwart einer Base, wie zum Beispiel Pyridin, hergestellt werden.

Die Einführung der Essigsäure-Gruppierung kann wie in WO 2006/005609 beschrieben in einer 4-Stufen-Sequenz (1. Substitution der Fluchtgruppe LG in IV mit dem Natriumsalz von Dimethylmalonat, 2. Verseifung der ersten Estergruppe, 3. Thermische Decarboxylierung, 4. Verseifung der zweiten Estergruppe) erfolgen.

Die Amin-Seitenkette X kann unter Verwendung der aus der Peptid-Chemie bekannten Standard-Kupplungsreagenzien, wie zum Beispiel EDC (N-(3-Dimethylaminopropyl)-N'-ethyl-carbodiimid-hydrochlorid) und HOBT (1-Hydroxy-benzotriazol) in einem Lösungsmittel, wie zum Beispiel N,N-Dimethylformamid, an die Carbonsäure VIII gekuppelt werden. Die Amine X können entweder käuflich erworben oder nach literaturbekannten Methoden hergestellt werden. Die Darstellung der erfindungsgemässen Verbindungen mit R³ = H kann durch Verwendung entsprechender Boc-geschützter Amine X (R³ = Boc) erfolgen. Die Boc-Schutzgruppe kann nach der Sulfonylierung entfernt werden, zum Beispiel durch Behandlung mit Trifluoressigsäure in Dichlormethan.

Die Sulfonylierung kann durch Deprotonierung der Kupplungsprodukte IX mit einer starken Base, wie zum Beispiel Natriumhydrid oder Kalium-*tert*-butylat, und anschließende Behandlung mit Sulfonsäurechloriden XI in einem Lösungsmittel, wie zum Beispiel DMF erfolgen und führt zu den erfindungsgemäßen Verbindungen I. Die eingesetzten Sulfonsäurechloride XI können entweder käuflich erworben oder in analoger Weise zu bekannten Verfahren (siehe z.B. J. Med. Chem. 40, 1149 (1997)) hergestellt werden. 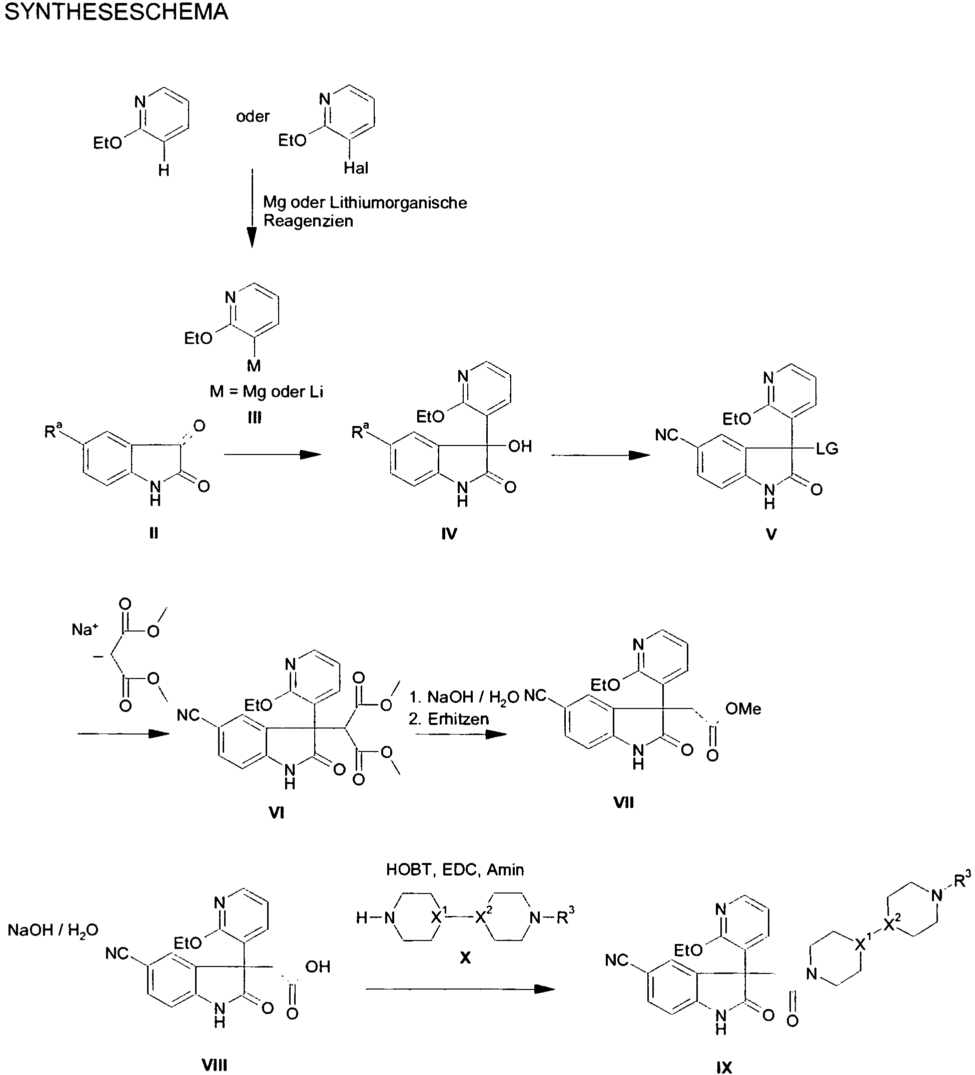 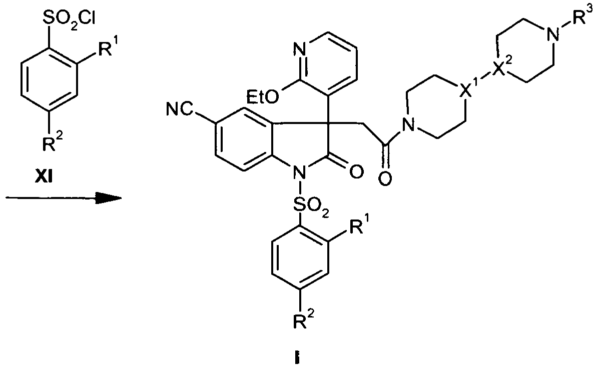 EtO = Ethoxy
R^{a} = CN oder I

Eine weitere Möglichkeit, die erfindungsgemäßen Verbindungen mit R³ = Methyl, Ethyl, n-Propyl oder Isopropyl herzustellen, ist die Umsetzung der sekundären Piperidin- bzw. Piperazin-Verbindungen I (R³ = H) mit Aldehyden oder Ketonen in Gegenwart von Reduktionsmitteln, wie zum Beispiel Natriumcyanoborhydrid oder Natriumacetoxyborhydrid, im Sinne einer reduktiven Aminierung dar (J. March, Advanced Organic Chemistry, 1992, 4. Auflage, Wiley, New York, Seiten 411; 898).

Ein weiterer Gegenstand der vorliegenden Erfindung betrifft ein pharmazeutisches Mittel, enthaltend mindestens eine Verbindung der allgemeinen Formel I und/oder ein pharmazeutisch verträgliches Salz davon, wie vorstehend ausgeführt, und einen pharmazeutisch verträglichen Träger. Geeignete Träger hängen unter anderem von der Darreichungsform des Mittels ab und sind dem Fachrpann grundsätzlich bekannt. Einige geeignete Träger sind weiter unten beschrieben.

Ein weiterer Gegenstand der vorliegenden Erfindung betrifft die Verwendung von Verbindungen der Formel I und/oder von pharmazeutisch verträglichen Salzen davon zur Herstellung eines Medikaments zur Behandlung und/oder Prophylaxe von Vasopressin-abhängigen Krankheiten.

Vasopressin-abhängige Krankheiten sind solche, bei denen der Krankheitsverlauf zumindest teilweise von Vasopressin abhängt, d.h. Krankheiten, die einen erhöhten Vasopressin-Spiegel zeigen, der unmittelbar oder indirekt zum Krankheitsbild beitragen kann. Anders ausgedrückt sind Vasopressin-abhängige Krankheiten solche, die durch Modulation des Vasopressinrezeptors, beispielsweise durch Gabe eines Vasopressinrezeptorliganden (Agonist, Antagonist, partieller Antagonist/Agonist, inverser Agonist etc.), beeinflusst werden können.

In einer bevorzugten Ausführungsform betrifft die vorliegende Erfindung die Verwendung von erfindungsgemäßen Verbindungen der Formel I oder von pharmazeutisch verträglichen Salzen zur Herstellung eines Medikaments zur Behandlung und/oder Prophylaxe von Krankheiten, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion. Unter dem Begriff "Diabetes" sind alle Diabetesformen zu verstehen, vor allem Diabetes mellitus (einschließlich Typ I und insbesondere Typ 11), Diabetes renalis und insbesondere Diabetes insipidus. Bevorzugt handelt es sich bei den Diabetesformen um Diabetes mellitus vom Typ II (mit Insulinresistenz) oder Diabetes insipidus.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Krankheiten, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, und Reisekrankheit.

Die erfindungsgemäßen Verbindungen der Formel I bzw. ihre pharmazeutisch verträglichen Salze oder das erfindungsgemäße pharmazeutische Mittel können auch zur Behandlung von verschiedenen Vasopressin-abhängigen Beschwerden, die zentralnervöse Ursachen oder Veränderungen in der HPA-Achse (hypothalamic pituitary adrenal axis) aufweisen, verwendet werden, zum Beispiel bei affektiven Störungen, wie depressiven Störungen und bipolaren Störungen. Dazu gehören zum Beispiel dysthyme Störungen, Phobien, posttraumatische Belastungsstörungen, generelle Angsstörungen, Panikstörungen, saisonale Depressionen und Schlafstörungen.

Ebenso können die erfindungsgemäßen Verbindungen der Formel I bzw. ihre pharmazeutisch verträglichen Salze oder das erfindungsgemäße pharmazeutische Mittel zur Behandlung bei Angststörungen und stressabhängigen Angststörungen eingesetzt werden, wie zum Beispiel generalisierten Angststörungen, Phobien, posttraumatischen Angststörungen, panischen Angststörungen, obsessiv-zwanghaften Angststörungen, akuten stressabhängigen Angststörungen und Sozialphobie.

Weiterhin können die erfindungsgemäßen Verbindungen auch zur Anwendung in einem Verfahren zur Behandlung von Gedächnisleistungsstörungen, Morbus Alzheimer, Psychosen, psychotischen Störungen, Schlafstörungen und/oder dem Cushing Syndrom sowie allen stressabhängigen Krankheiten eingesetzt werden.

Dementsprechend betrifft eine weitere bevorzugte Ausführungsform der vorliegenden Erfindung die Verwendung von erfindungsgemäßen Verbindungen der Formel I oder von pharmazeutisch verträglichen Salzen davon zur Herstellung eines Medikaments zur Behandlung von affektiven Störungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Angststörungen und/oder stressabhängigen Angststörungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Gedächtnisleistungsstörungen und/oder Morbus Alzheimer.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Psychosen und/oder psychotischen Störungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung des Cushing-Syndroms oder sonstigen stressabhängigen Krankheiten.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Schlafstörungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von depressiven Erkrankungen. Eine besondere Form von depressiven Erkrankungen sind sogenannte childhood onset mood disorders, d.h. depressive Verstimmungen, die bereits in der Kindheit einsetzen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von vasomotorischen Symptomen und/oder thermoregulatorischen Fehlfunktionen, wie beispielsweise das "hot flush"-Symptom.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Drogen-, Arzneimittel- und/oder durch sonstige Faktoren vermittelten Abhängigkeiten, zur Behandlung und/oder Prophylaxe von Stress bedingt durch den Entzug von einem oder mehreren die Abhängigkeit vermittelnden Faktoren und/oder zur Behandlung und/oder Prophylaxe von Stress-induzierten Rückfällen in die Drogen-, Arzneimittel- und/oder durch sonstige Faktoren vermittelten Abhängigkeiten.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Schizophrenie und/oder Psychose.

Bei dem mit dem Verfahren prophylaktisch oder therapeutisch zu behandelnden Patienten handelt es sich vorzugsweise um ein Säugetier, beispielsweise um einen Menschen oder um ein nichtmenschliches Säugetier oder um ein nichtmenschliches transgenes Säugetier. Speziell handelt es sich um einen Menschen.

Die Verbindungen der allgemeinen Formel ihrer pharmazeutisch verträglichen Salze wie vorstehend ausgeführt, können von einem Fachmann in Kenntnis der erfindungsgemäßen technischen Lehre in Ausführung und/oder in analoger Ausführung von an sich bekannten Verfahrensschritten hergestellt werden.

Die Verbindungen I bzw. deren pharmazeutisch verträglichen Salze zeichnen sich dadurch aus, dass sie eine Selektivität zum Vasopressin-Rezeptorsubtyp V1 b gegenüber mindestens einem der nahe verwandten Vasopressin/Oxytozin-Rezeptorsubtypen (zum Beispiel Vasopressin V1 a, Vasopressin V2 und/oder Oxytozin) aufweisen.

Alternativ oder vorzugsweise zusätzlich zeichnen sich die Verbindungen I bzw. deren pharmazeutisch verträglichen Salze dadurch aus, dass sie eine verbesserte metabolische Stabilität aufweisen.

Die metabolische Stabilität einer Verbindung kann beispielsweise gemessen werden, indem man eine Lösung dieser Verbindung mit Lebermikrosomen von bestimmten Spezies (zum Beispiel Ratte, Hund oder Mensch) inkubiert und die Halbwertszeit der Verbindung unter diesen Bedingungen bestimmt (RS Obach, Curr Opin Drug Discov Devel. 2001, 4, 36-44). Dabei kann aus einer beobachteten größeren Halbwertszeit auf eine verbesserte metabolische Stabilität der Verbindung geschlossen werden. Die Stabilität in Gegenwart von humanen Lebermikrosomen ist von besonderem Interesse, da sie eine Vorhersage für den metabolischen Abbau der Verbindung in der menschlichen Leber ermöglicht. Verbindungen mit erhöhter metabolischer Stabilität (gemessen in dem Lebermikrosomen-Test) werden deshalb wahrscheinlich auch in der Leber langsamer abgebaut. Der langsamere metabolische Abbau in der Leber kann zu höheren und/oder länger anhaltenden Konzentrationen (Wirkspiegel) der Verbindung im Körper führen, so dass die Eliminierungs-Halbwertszeit der erfindungsgemässen Verbindungen erhöht ist. Erhöhte und/oder länger anhaltende Wirkspiegel können zu einer besseren Wirksamkeit der Verbindung in der Behandlung oder Prophylaxe von verschiedenen Vasopressin-abhängigen Krankheiten führen. Außerdem kann eine verbesserte metabolische Stabilität zu einer erhöhten Bioverfügbarkeit nach oraler Gabe führen, da die Verbindung nach erfolgter Resorption im Darm einem geringeren metabolischen Abbau in der Leber (sogenannter "first pass effect") unterliegt. Eine erhöhte orale Bioverfügbarkeit kann aufgrund erhöhter Konzentration (Wirkspiegel) der Verbindung zu einer besseren Wirksamkeit der Verbindung nach oraler Gabe führen.

Die erfindungsgemäßen Verbindungen sind nach Verabreichung auf verschiedenen Wegen wirksam. Die Verabreichung kann beispielsweise intravenös, intramuskulär, subkutan, topisch, intratracheal, intranasal, transdermal, vaginal, rektal, sublingual, bukkal oder oral erfolgen und erfolgt häufig intravenös, intramuskulär oder insbesondere oral.

Die vorliegende Erfindung betrifft auch pharmazeutische Zusammensetzungen, die eine wirksame Dosis einer erfindungsgemäßen Verbindung I oder eines pharmazeutisch verträglichen Salzes davon und geeignete pharmazeutische Träger (Arzneiträger) enthalten.

Diese Arzneiträger werden entsprechend der pharmazeutischen Form und der gewünschten Applikationsart gewählt und sind dem Fachmann grundsätzlich bekannt.

Die erfindungsgemäßen Verbindungen der Formel I oder gegebenenfalls geeignete Salze dieser Verbindungen können zur Herstellung von pharmazeutischen Zusammensetzungen zur oralen, sublingualen, bukkalen, subkutanen, intramuskulären, intravenösen, topischen, intratrachealen, intranasalen, transdermalen, vaginalen oder rektalen Verabreichung verwendet werden und Tieren oder Menschen in einheitlichen Verabreichungsformen, gemischt mit herkömmlichen pharmazeutischen Trägern, zur Prophylaxe oder Behandlung der obigen Störungen oder Krankheiten verabreicht werden.

Die geeigneten Verabreichungsformen (Dosiereinheiten) umfassen Formen zur oralen Verabreichung, wie Tabletten, Gelatinekapseln, Pulver, Körnchen und Lösungen oder Suspensionen zur oralen Einnahme, Formen zur sublingualen, bukkalen, intratrachealen oder intranasealen Verabreichung, Aerosole, Implantate, Formen der subkutanen, intramuskulären oder intravenösen Verabreichung und Formen der rektalen Verabreichung. Zur topischen Verabreichung können die erfindungsgemäßen Verbindungen in Cremes, Salben oder Lotionen verwendet werden.

Um den gewünschten prophylaktischen oder therapeutischen Effekt zu erzielen, kann die Dosis des Wirkstoffs zwischen 0.01 und 50 mg pro kg Körpergewicht und pro Tag variieren.

Jede Einheitsdosis kann 0.05 bis 5000 mg, vorzugsweise 1 bis 1000 mg, des Wirkstoffs in Kombination mit einem pharmazeutischen Träger enthalten. Diese Einheitsdosis kann 1-bis 5-mal am Tag verabreicht werden, so dass eine tägliche Dosis von 0,5 bis 25000 mg, vorzugsweise 1 bis 5000 mg, verabreicht wird.

Falls eine feste Zusammensetzung in Form von Tabletten zubereitet wird, wird der Wirkstoff mit einem festen pharmazeutischen Träger, wie Gelatine, Stärke, Laktose, Magnesiumstearat, Talk, Siliziumdioxid oder Ähnlichem, gemischt.

Die Tabletten können mit Saccharose, einem Cellulosederivat oder einer anderen geeigneten Substanz beschichtet werden oder anders behandelt werden, um eine anhaltende oder verzögerte Aktivität aufzuweisen und um eine vorbestimmte Menge des Wirkstoffs kontinuierlich freizugeben.

Eine Zubereitung in Form von Gelatinekapseln wird durch Mischen des Wirkstoffs mit einem Streckmittel und Aufnehmen der resultierenden Mischung in weiche oder harte Gelatinekapseln erhalten.

Eine Zubereitung in Form eines Sirups oder Elixiers oder zur Verabreichung in Form von Tropfen kann Wirkstoffe zusammen mit einem Süßstoff, der vorzugsweise kalorienfrei ist, Methylparaben oder Propylparaben als Antiseptika, einen Aromastoff und einen geeigneten Farbstoff enthalten.

Die wasserdispergierbaren Pulver oder Körnchen können die Wirkstoffe, gemischt mit Dispergiermitteln, Benetzungsmitteln oder Suspensionsmitteln, wie Polyvinylpyrrolidone, sowie Süßstoffe oder Geschmackskorrigentien, enthalten.

Eine rektale oder vaginale Verabreichung wird durch Verwendung von Zäpfchen erreicht, die mit Bindemitteln zubereitet werden, die bei rektaler Temperatur schmelzen, zum Beispiel Kakaobutter oder Polyethylenglykole. Eine parenterale Verabreichung erfolgt unter Verwendung von wässrigen Suspensionen, isotonischen Salzlösungen oder sterilen und injizierbaren Lösungen, die pharmakologisch verträgliche Dispergiermittel und/oder Benetzungsmittel, zum Beispiel Propylenglykol oder Polyethylenglykol, enthalten.

Der Wirkstoff kann auch als Mikrokapseln oder Zentrosome, falls geeignet mit einem oder mehreren Trägern oder Additiven, formuliert werden.

Zusätzlich zu den erfindungsgemäßen Verbindungen können die erfindungsgemäßen Mittel andere Wirkstoffe enthalten, die zur Behandlung der oben angegebenen Störungen oder Krankheiten nützlich sein können.

Die vorliegende Erfindung betrifft somit weiterhin pharmazeutische Mittel, in denen mehrere Wirkstoffe zusammen anwesend sind, wobei mindestens einer von diesen eine erfindungsgemäße Verbindung I oder Salz davon ist.

Die Erfindung wird nachstehend anhand von Beispielen näher erläutert, wobei die Beispiele nicht einschränkend zu verstehen sind.

Die Herstellung der erfindungsgemäßen Verbindungen kann über verschiedene Syntheserouten erfolgen. Die genannten Vorschriften, wie entsprechend im Syntheseschema beschrieben, sind nur exemplarisch anhand der genannten Beispiele näher erläutert, ohne ausschließlich auf den genannten Syntheseweg oder Analogvorschriften beschränkt zu sein.

### EXPERIMENTELLER TEIL

Verwendete Abkürzungen:
- THF:: Tetrahydrofuran
- DMSO:: Dimethylsulfoxid
- TFA:: Trifluoressigsäure
- p:: pseudo (beispielsweise pt pseudo Triplett)
- b:: breit (beispielsweise bs breites Singulett)
- s:: Singulett
- d:: Doublett
- t:: Triplett
- m:: Multiplett
- dd:: doppeltes Doublett
- dt:: doppeltes Triplett
- tt:: dreifaches Triplett

### I. Herstellung der Ausgangsverbindung X

### a) 1-Ethyl-4-piperidin-4-yl-piperazin

### a.1 tert-Butyl 4-(4-ethylpiperazin-1-yl)piperidin-1-carboxylat

29.2 g (256 mmol) *N*-Ethylpiperazin wurden mit 50.0 g (256 mmol) *tert*-Butyl 4-oxopiperidin-1-carboxylat (entspricht 1-Boc-4-piperidon) unter Eiskühlung in 800 ml Ethanol vorgelegt. Man gab 15.4 g (256 mmol) Eisessig zu. Anschließend gab man portionsweise 16.1 g (256 mmol) Natriumacetoxyborhydrid zur gekühlten Reaktionsmischung. Zunächst war eine leichte Gasbildung und nach 2/3-Zugabe des Reduktionsmittels eine Schaumbildung zu beobachten. Die Reaktionsmischung wurde über Nacht bei Raumtemperatur gerührt. Zur Aufarbeitung wurde die Reaktionslösung unter Kühlung mit 200 ml 2 *N* Natronlauge versetzt, das Lösungsmittel Ethanol abdestilliert und die übrigbleibende Reaktionsmischung mit Wasser verdünnt. Man extrahierte mit Diethylether (2 x), wusch mit gesättigter Natriumchlorid-Lösung (1 x), trocknete die vereinigten organischen Phasen über Magnesiumsulfat, filtrierte und entfernte das Lösungsmittel im Vakuum. Die rohe Titelverbindung wurde als gelbes Öl erhalten, das anschließend über eine mit Kieselgel gefüllte, 4 I-Nutsche mit Dichlormethan und 10 % Methanol als Elutionsmittel chromatographiert wurde. Es wurden insgesamt 40 g (135 mmol, 53 %) *tert*-Butyl 4-(4-ethylpiperazin-1-yl)piperidin-1-carboxylat erhalten.

### a.2 1-Ethyl-4-piperidin-4-yl-piperazin als Chlorid-Salz

Zur Schutzgruppenentfernung wurden 40 g (135 mmol) *tert*-Butyl 4-(4-ethylpiperazin-1-yl)piperidin-1-carboxylat aus Beispiel a.1 in 200 ml Methanol und 1.8 I Dichlormethan vorgelegt und mit 100 ml 5-6 *M* HCl-Lösung in Isopropanol versetzt. Es entstand eine Suspension, wobei auch eine leichte Gasentwicklung zu beobachten war. Der Reaktionsansatz wurde eine Stunde bei 40 °C (Wasserbadtemperatur) gerührt und 48 Stunden bei Raumtemperatur nachgerührt. Zur vollständigen Entschützung wurden nochmals 50 ml der 5-6 *M* HCl-Lösung in Isopropanol zugegeben und die Reaktionsmischung bei 40 °C gerührt. Am Rotationsverdampfer wurde das Dichlormethan abdestilliert. Es wurden nochmals 200 ml Methanol und 30 ml der 5-6 *M* HCl-Lösung in Isopropanol zugesetzt. Man rührte die Reaktionsmischung eine Stunde am Rückfluss, wobei sich eine weiße Suspension mit starker Gasentwicklung bildete. Danach entstand eine dünnflüssige Suspension, die auf Raumtemperatur abgekühlt wurde. Der Niederschlag wurde abgesaugt und mit Methanol und Diethylether gewaschen. Nach dem Trocknen wurden 36 g (117 mmol, 87 %) des 1-Ethyl-4-piperidin-4-yl-piperazins als Chlorid-Salz isoliert.
¹H-NMR (D₂O, 400 MHz) δ [ppm] = 3.74 - 3.47 (m, 11H), 3.28 (q, 2H, J = 7.3 Hz), 3.06 (dt, 2H, J = 2.2 Hz, J = 13.2 Hz), 2.38 (m, 2H, J = 13.6 Hz), 1.89 (dq, 2H, J = 4.1 Hz, J = 13.3 Hz), 1.30 (t, 3H, J = 7.3 Hz).

### II. Herstellung der racemischen Verbindungen der Formel I

### BEISPIEL 1

### (±)-1-Phenylsulfonyl-3-(2-ethoxy-pyridin-3-yl)-3-(2-[4-(4-methyl-piperazin-1-yl)-piperidin-1-yl]-2-oxo-ethyl}-2-oxo-2,3-dihydro-1H-indol-5-carbonitril, Trifluoressigsäure-Salz (Verbindung I-34)

### 1.1 3-(2-Ethoxypyridin-3-yl)-3-hydroxy-5-iod-1,3-dihydro-2H-indol-2-on

Zu 20.86 g (76.40 mmol) 5-lodoisatin in 400 ml wasserfreiem Tetrahydrofuran (THF) gab man unter Kühlung mit einem Eisbad und Rühren 3.22 g (80.50 mmol, 60%w/w) Natriumhydrid portionsweise zu, so dass die Temperatur zwischen 0-10 °C gehalten wurde. Die Suspension wurde unter Eisbadkühlung eine Stunde gerührt.

Zur Herstellung des Pyridin-Grignards löste man bei Raumtemperatur 20 g (80.30 mmol) 2-Ethoxy-3-iod-pyridin in 400 ml wasserfreiem THF. Man gab zu dieser Lösung unter Kühlung 95.6 ml (1 M Lösung in THF, 95.60 mmol) Ethylmagnesiumbromid innerhalb von 5-10 Minuten bei einer Temperatur zwischen 22 und 15 °C. Die Lösung wurde 20 Minuten gerührt, wobei sie sich von farblos zu leicht gelblich färbte.

Die Lösung des Pyridin-Grignards wurde anschließend zur Eisbad-gekühlten Lösung des 5-lodisatin-Natrium-Salzes innerhalb von 5-10 Minuten bei einer Temperatur zwischen 5 und 18 °C gegeben. Nach beendeter Zugabe des Pyridin-Grignards wurde das Eisbad entfernt. Die Reaktionsmischung wurde bei Raumtemperatur 2 Stunden nachgerührt. Ein Überschuss an gesättigter Ammoniumchlorid-Lösung wurde zugegeben, gefolgt von Ethylacetat. Die Mischung wurde weitere 5 Minuten gerührt. Die wässrige Phase wurde abgetrennt und mit Ethylacetat (2 x) extrahiert. Die vereinigten organischen Phasen wurden mit Wasser (2 x) gewaschen und das Lösungsmittel im Vakuum entfernt. Dabei fiel aus der noch verdünnten Lösung zuerst unreagiertes 5-Iodisatin aus und wurde abgetrennt. Nach dem weiteren Aufkonzentrieren kristallisierte schließlich auch die Titelverbindung aus. Die Suspension wurde im Kühlschrank zwei Stunden bei 5 °C gelagert. Der ausgefallene, schwach gelbe Feststoff wurde anschließend abfiltriert und mit wenig Ethylacetat nachgewaschen. Nach dem Trocknen bei 40 °C erhielt man die Titelverbindung (17.1 g, 43.16 mmol, 57 %).
ESI-MS [M+H⁺] = 397 Berechnet für C₁₅H₁₃IN₂O₃ = 396.19

### 1.2 5-Cyano-3-hydroxy-3-(2-ethoxypyridin-3-yl)-1,3-dihydroindol-2-on

7.1 g (17.92 mmol) 3-(2-Ethoxypyridin-3-yl)-3-hydroxy-5-iod-1,3-dihydro-2*H*-indol-2-on in 100 ml wasserfreiem THF wurden unter Stickstoffatmosphäre bei Raumtemperatur gerührt. Man gab 2.1 g (17.92 mmol) Zinkcyanid zu, gefolgt von 0.51 g (0.45 mmol) des Katalysators Tetrakis(triphenylphosphin)-palladium(0). Die Reaktionsmischung wurde direkt in ein vorgeheiztes Ölbad mit einer Temperatur von 100 °C gestellt. Man rührte bei 100 °C (Ölbadtemperatur) und gab nach 30 Minuten weitere 0.51 g (0.45 mmol) des Katalysators zu. Insgesamt wurde für 2 Stunden gerührt. Man ließ die Reaktionsmischung auf Raumtemperatur abkühlen und gab einen Überschuss an Wasser zu. Anschließend extrahierte man mit Ethylacetat (3 x) und wusch die vereinigten organischen Phasen dreimal mit Wasser. Das Lösungsmittel wurde im Vakuum bis zur Trockene evaporiert und der Rückstand mit kleinen Volumina an Ethylacetat aufgeschlämmt. Man filtrierte einen schwach gelben Feststoff ab, der mit Ethylacetat nachgewaschen und im Vakuumtrockenschrank getrocknet wurde. Man erhielt 3.7 g (12.44 mmol, 69.4%) der Titelverbindung.
ESI-MS [M+H⁺] = 296 Berechnet für C₁₆H₁₃N₃O₃ = 295.30

### 1.3 3-Chlor-3-(2-ethoxypyridin-3-yl)-2-oxoindolin-5-carbonitril

Zu einer Suspension von 6.00 g (20.32 mmol) 5-Cyano-3-hydroxy-3-(2-ethoxypyridin-3-yl)-1,3-dihydroindol-2-on aus Beispiel 1.2 in 60 ml wasserfreiem Dichlormethan (getrocknet über Molekularsieb) gab man unter Stickstoff 2.30 ml (28.45 mmol) Pyridin. Nach dem Abkühlen der Reaktionsmischung auf 0 °C tropfte man 2.06 ml (28.45 mmol) Thionylchlorid in Substanz zu (exotherme Reaktion) und rührte danach die Reaktionsmischung eine Stunde bei Raumtemperatur. Es entstand eine gelbe Suspension. Der Reaktionsverlauf wurde mittels Dünnschichtchromatographie (TLC) (Kieselgel, Dichlormethan / Methanol im Verhältnis 95 : 5) verfolgt. Die Reaktionsmischung wurde vorsichtig in Eiswasser gegossen. Nach 15 Minuten Rühren wurde die organische Phase abgetrennt. Die wässrige Phase wurde mit Dichlormethan (2 x) extrahiert. Alle organischen Phasen wurden vereinigt und über Magnesiumsulfat getrocknet, filtriert und das Lösungsmittel im Vakuum entfernt. Man erhielt 5.70 g (18.17 mmol, 89 %) der Titelverbindung als amorphen Feststoff, der ohne weitere Aufreinigung in der nächsten Stufe eingesetzt wurde.

### 1.4 Dimethyl [5-cyano-3-(2-ethoxypyridin-3-yl)-2-oxo-2,3-dihydro-1H-indol-3-yl]malonat

Zu einer auf 10 °C gekühlten Suspension von 1.808 g (45.23 mmol, 60%w/w) Natriumhydrid in 100 ml Dimethylformamid tropfte man langsam 5.68 ml (49.75 mmol) Dimethylmalonat. Anschließend rührte man die Reaktionsmischung 30 Minuten bei Raumtemperatur und gab danach 4.73 g (15.08 mmol) 3-Chlor-3-(2-ethoxypyridin-3-yl)-2-oxoindolin-5-carbonitril aus Beispiel 1.3 portionsweise in Substanz zu. Die Reaktionslösung färbte sich dunkelrot und wurde noch weitere 15 Minuten bei Raumtemperatur gerührt. Der Reaktionsverlauf wurde dünnschichtchromatographisch kontrolliert (Kieselgel, Heptan / Ethylacetat 1 : 1). Der Ansatz wurde zur Aufarbeitung in kalte 1 N HCl eingerüht und mit Dichlormethan versetzt. Die Phasen wurden getrennt, und die wässrige Phase mit Dichlormethan (1 x) extrahiert. Die vereinigte organische Phase wurde zunächst mit Wasser (1 x) und anschließend mit gesättigter Natriumchlorid-Lösung (1 x) gewaschen, über Magnesiumsulfat getrocknet, filtriert und das Lösungsmittel im Vakuum entfernt. Der Rückstand wurde erneut in etwas Dichormethan angelöst und mit Pentan versetzt. Es fielen mehrere Fraktionen Kristallisat an. Man erhielt insgesamt 6.48 g (15.83 mmol, 100 %) der Titelverbindung als gelben Feststoff, der nur geringfügig verunreinigt war.
ESI-MS [M+H⁺] = 410 Berechnet für C₂₁H₁₉N₃O₆ = 409.40

### 1.5 [5-Cyano-3-(2-ethoxypyridin-3-yl)-2-oxo-2,3-dihydro-1H-indol-3-yl]essigsäuremethylester

Zu einer Lösung von 6.48 g (15.83 mmol) Dimethyl [5-cyano-3-(2-ethoxypyridin-3-yl)-2-oxo-2,3-dihydro-1*H*-indol-3-yl]malonat aus Beispiel 1.4 in 6 ml Ethanol gab man 60 ml 2 N Natronlauge und rührte 1 Stunde bei Raumtemperatur. Danach konnte gemäß DC-Kontrolle (Kieselgel, Heptan / Ethylacetat 1 : 1) kein Edukt mehr nachgewiesen werden. Die Reaktionsmischung wurde in eiskalte 1 N Salzsäure eingerührt und mit Dichlormethan versetzt. Man trennte die Phasen und extrahiert die wässrige Phase einmal mit Dichlormethan. Die vereinigte organische Phase wurde zunächst mit Wasser (1 x) und anschließend mit gesättigter Natriumchlorid-Lösung (1 x) gewaschen, über Magnesiumsulfat getrocknet, filtriert und das Lösungsmittel im Vakuum entfernt. Der erhaltene gelbliche Feststoff (5.46 g) wurde im Vakuumtrockenschrank bei 40 °C getrocknet.

Der erhaltene gelbliche Feststoff (5.46 g) wurde in einem mit Stickstoff inertisierten Einhalskolben auf 150 °C erwärmt. Dabei bildete sich unter CO₂-Gasentwicklung das Methyl [5-cyano-3-(2-ethoxypyridin-3-yl)-2-oxo-2,3-dihydro-1*H*-indol-3-yl]acetat. Der Reaktionsansatz wurde auf Raumtemperatur abgekühlt, und die Substanz mit Methanol versetzt. Es bildete sich ein Kristallisat, das im Kühlschrank bei 5 °C über Nacht aufbewahrt wurde. Der Feststoff wurde abgesaugt und mit mit wenig Methanol gewaschen. Man erhielt 2.2 g (5.95 mmol, 38 %, 95 % Reinheit) der Titelverbindung als beige-farbener Feststoff.
ESI-MS [M+H⁺] = 352 Berechnet für C₁₉H₁₇N₃O₄ = 351.37

### 1.6 [5-Cyano-3-(2-ethoxypyridin-3-yl)-2-oxo-2,3-dihydro-1H-indol-3-yl]essigsäure

Zu einer Lösung von 1.557 g (4.43 mmol) 5-Cyano-3-(2-ethoxypyridin-3-yl)-2-oxo-2,3-dihydro-1*H*-indol-3-yl]essigsäuremethylester aus Beispiel 1.5 in 6 ml Ethanol gab man zunächst 15 ml Wasser und anschließend 9 ml 2 N Natronlauge. Man rührte die Reaktionsmischung 5 Stunden bei Raumtemperatur. Laut TLC-Kontrolle (Kieselgel, Dichlormethan / Methanol 9 : 1) war die Reaktion vollständig erfolgt. Zur Aufarbeitung wurde die Reaktionsmischung mit 1 N Salzsäure auf pH 5 gestellt und anschließend am Rotationsverdampfer eingeengt. Der Rückstand wurde in Toluol aufgenommen und erneut bis zur Trockene eingeengt. Der erhaltene beige-farbene Feststoff wurde im Vakuumtrockenschrank bei 40 °C getrocknet. Man erhielt 2.70 g (4.43 mmol, 55 % NaCl-Salzgehalt, 99 % d.Th. Ausbeute) der Titelverbindung .
ESI-MS [M+H⁺] = 338 Berechnet für C₁₈H₁₅N₃O₄ = 337.34

### 1.7 3-(2-Ethoxypyridin-3-yl)-3-{2-[4-(4-methyl-piperazin-1-yl)-piperidin-1-yl]-2-oxoethyl}-2-oxoindolin-5-carbonitril

Zu einer Lösung von 907 mg (1.48 mmol) [5-Cyano-3-(2-ethoxypyridin-3-yl)-2-oxo-2,3-dihydro-1*H*-indol-3-yl]essigsäure aus Beispiel 1.6 in 10 ml Dimethylformamid gab man 200 mg (1.48 mmol) 1-Hydroxybenzotriazol und 284 mg (1.48 mmol) N-(3-Dimethylaminopropyl)-N'-ethyl-carbodiimid-hydrochlorid. Man rührte 15 Minuten und gab anschließend 285 mg (1.56 mmol) 1-Methyl-4-piperidin-4-yl-piperazin und 1.03 ml (7.41 mmol) Triethylamin zu. Die Reaktionsmischung wurde bei Raumtemperatur über Nacht gerührt. Man kontrolliete die Reaktion mit TLC (Kieselgel, Dichlormethan / Methanol 15 : 5). Der Reaktionsansatz wurde mit Wasser versetzt und mit Ethylacetat verdünnt. Die Phasen wurden getrennt und die wässrige Phase mit Ethylacetat extrahiert (1 x). Die vereinigte organische Phase wurde noch mit Wasser (1 x) und gesättigter Natriumchlorid-Lösung (1 x) gewaschen, über Magnesiumsulfat getrocknet, filtriert und das Lösungsmittel im Vakuum verdampft. Der erhaltene Rückstand wurde über präparative MPLC (ISCO Companion, 12 g NP-Kartusche) mit Dichlormethan / Methanol (10 - 70 %) als Elutionsmittel gereinigt. Man erhielt 497 mg (0.99 mmol, 66 %) der Titelverbindung als farblosen Feststoff.
ESI-MS [M+H⁺] = 503 Berechnet für C₂₈H₃₄N₆O₃ = 502.62

### 1.8 1-Phenylsulfonyl-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril, Trifluoressigsäure-Salz

Zu einer auf 0 °C gekühlten Lösung von 80.0 mg (0.16 mmol) 3-(2-Ethoxypyridin-3-yl)-3-{2-[4-(4-methyl-piperazin-1-yl)-piperidin-1-yl]-2-oxoethyl}2-oxoindolin-5-carbonitril aus Beispiel 1.7 in 2 ml Dimethylformamid gab man 6.6 mg (0.19 mmol) Natriumhydrid und nach 10 Minuten 24 L (0.19 mmol) Phenylsulfonylchlorid. Den Reaktionsansatz ließ man auf Raumtemperatur erwärmen und rührte 30 Minuten nach. Der Reaktionsverlauf wurde dünnschichtchromatographisch verfolgt (Kieselgel, Dichlormethan / Methanol 1 : 1). Zu der Reaktionsmischung gab man gesättigte Natriumhydrogencarbonat-Lösung und Ethylacetat. Die beiden Phasen wurden anschließend getrennt und die wässrige Phase nochmals mit Ethylacetat (1 x) extrahiert. Die vereinigte organische Phase wurde mit Wasser (1 x) und mit gesättigter Natriumchlorid-Lösung (1 X) gewaschen, über Magnesiumsulfat getrocknet, filtriert und das Lösungsmittel im Vakuum evaporiert. Der Rückstand wurde über präparative HPLC (RP, Eluenten Acetonitril / Wasser, 0.01 % TFA) gereinigt. Man erhielt 54.4 mg (0.08 mmol, 51 %, 95 % Reinheit) der Titelverbindung als farblosen Feststoffs.
ESI-MS [M+H⁺] = 643 Berechnet für C₃₄H₃₈ClN₆O₅S = 642.78

Die Verbindungen der Formel I gemäß den Beispielen 2 bis 9 können unter Verwendung der entsprechenden Ausgangsverbindungen analog zum Herstellungsverfahren des Beispiels 1 hergestellt werden.

Die erfindungsgemäßen Verbindungen I können beispielsweise durch Kristallisation, herkömmliche Säulenchromatographie über Normalphase (wie beispielsweise NP-SiO₂-Kartusche, Chromabond und Dichlormethan / Methanol als Eluenten) und / oder durch präparative HPLC (RP, Eluenten Acetonitril / Wasser, 0.1 % TFA oder 0.1 % Essigsäure) gereinigt werden. Die Verbindungen I fallen dann als Trifluoressigsäure-Salz bzw. Essigsäure-Salz an:

### BEISPIEL 2

### (±)-1-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril Trifluoressigsäure-Salz (Verbindung I-37)

ESI-MS [M+H⁺] = 717 Berechnet für C₃₇H₄₄ClN₆O₇S = 716.86

### BEISPIEL 3

### (±)-1-Phenylsulfonyl-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril, Tritluoressigsäure-Salz (Verbindung I-40)

ESI-MS [M+H⁺] = 657 Berechnet für C₃₅H₄₀ClN₆O₅S = 656.81

### BEISPIEL 4

### (±)-3-(2-Ethoxy-pyridin-3-yl)-3-{2-[4-(4-ethyl-piperazin-1-yl)-piperidin-1-yl]-2-oxo-ethyl}-1-(2-methoxy-phenylsulfonyl)-2-oxo-2,3-dihydro-1H-indol-5-carbonitril, Trifluoressigsäure-Salz (Verbindung I-38)

ESI-MS [M+H⁺] = 687 Berechnet für C₃₆H₄₂ClN₆O₆S = 686.84

### BEISPIEL 5

### (±)-3-(2-Ethoxy-pyridin-3-yl)-1-(2-methoxy-phenylsulfonyl)-3-{2-[4-(4-methyl-piperazin-1-yl)-piperidin-1-yl]-2-oxo-ethyl}-2-oxo-2,3-dihydro-1H-indol-5-carbonitril, Trifluoressigsäure-Salz (Verbindung I-32)

ESI-MS [M+H⁺] = 673 Berechnet für C₃₅H₄₀ClN₆O₆S = 672.81

### BEISPIEL 6

### (±)-1-(2,4-Dimethoxy-phenylsulfonyl)-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril, Trifluoressigsäure-Salz (Verbindung I-31)

ESI-MS [M+H⁺] = 703 Berechnet für C₃₆H₄₂N₆O₇S = 702.84

### BEISPIEL 7

### (±)-1-Phenylsulfonyl-3-(2-ethoxy-pyridin-3-yl)-3-{2-[4-(1-methyl-piperidin-yl)-piperazin-1-yl]-2-oxo-ethyl}-2-oxo-2,3-dihydro-1H-indol-5-carbonitril, Trifluoressigsäure-Salz (Verbindung I-4)

ESI-MS [M+H⁺] = 643 Berechnet für C₃₄H₃₈ClN₆O₅S = 642.78

### BEISPIEL 8

### (±)3-(2-Ethoxy-pyridin-3-yl)-1-(2-methoxy-phenylsulfonyl)-3-{2-[4-(1-methyl-piperidin-4-yl)-piperazin-1-yl]-2-oxo-ethyl}-2-oxo-2,3-dihydro-1H-indol-5-carbonitril, Trifluoressigsäure-Salz (Verbindung I-2)

ESI-MS [M+H⁺] = 673 Berechnet für C₃₅H₄₀ClN₆O₆S = 672.81

### BEISPIEL 9 (±)-1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril, Trifluoressigsäure-Salz

### (Verbindung I-1)

ESI-MS [M+H⁺] = 703 Berechnet für C₃₆H₄₂ClN₆O₇S = 702.84

### III. Herstellung von chiralen Verbindungen der allgemeinen Formel I

Die Trennung von racemischen Verbindungen der Formel I kann beispielsweise durch Separation auf einer präparativen chiralen Säule erfolgen.

### BEISPIEL 2A UND BEISPIEL 2B:

### Racematspaltung von (±)-1-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril, Trifluoressigsäure-Salz (Verbindung I-37)

219 mg (±)-1-(2,4-Dimethoxy-phenylsulfonyl)-3-(2-ethoxy-pyridin-3-yl)-3-}-2-[4-(4-ethylpiperazin-1-yl)-piperidin-1-yl]-2-oxo-ethyl}-2-oxo-2,3-dihydro-1H-indol-5-carbonitril, Trifluoressigsäure-Salz aus Beispiel 2 wurden über eine chirale präparative Säule (Chiralcell OD, Fluss 55 ml/min) mit *n*-Heptan / Ethanol (7 : 3) als Elutionsmittel getrennt. Man erhielt 41 mg des zuerst eluierenden Enantiomers mit positivem Drehwert (Beispiel 2A) und 25 mg des später eluierenden Enantiomers mit negativem Drehwert (Beispiel 2B).

### Beispiel 2A:

### (+)-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril (Verbindung I-37A)

ESI-MS [M+H⁺] = 717
HPLC (Chiralcel OD 0.46 cm x 25 cm ; n-Heptan / Ethanol 7:3) R_{f} = 11.1 min
[α]²⁰_{D} = +13 (c 0.1, CHCl₃)
¹H-NMR ([d₆]-DMSO, 500 MHz) δ [ppm] = 8.10 (1H), 7.95 (2H), 7.85 (1H), 7.80 (1H), 7.60 (1H), 7.00 (1H), 6.65 (2H), 4.10 (2H), 3.80-4.05 (6H), 3.60 (3H), 3.15-3.30 (1H), 2.90 (1H), 2.25-2.45 (11H), 1.70 (1H), 1.55 (1H), 1.25 (2H), 1.00-1.10 (3H), 0.98 (3H), 0.80 (1H).

### Beispiel 2B:

### (-)-(2,4-Dimethoxy-phenylsulfonyl)-3-(2-ethoxy-pyridin-3-yl)-3-{2-[4-(4-ethyl-piperazin-1-yl)-piperidin-1-yl]-2-oxo-ethyl}-2-oxo-2,3-dihydro-1H-indol-5-carbonitril (Verbindung I-37B)

ESI-MS [M+H⁺] = 717
HPLC (Chiralcel OD 0.46 cm x 25 cm ; n-Heptan / Ethanol 7:3) R_{f} = 15.9 min
[α]₂₀^{D}= -14 (c 0.1, CHCl₃)
¹H-NMR ([d₆]-DMSO, 500 MHz) δ [ppm] = 8.10 (1H), 7.95 (2H), 7.85 (1H), 7.80 (1H), 7.60 (1H), 7.00 (1H), 6.65 (2H), 4.10 (2H), 3.80-4.05 (6H), 3.60 (3H), 3.15-3.30 (1H), 2.90 (1H), 2.25-2.45 (11H), 1.70 (1H), 1.55 (1H), 1.25 (2H), 1.00-1.10 (3H), 0.98 (3H), 0.80 (1H).

### BEISPIEL 6A UND BEISPIEL 6B:

Racematspaltung von 1-(2,4-Dimethoxy-phenylsulfonyl)-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril, Trifluoressigsäure-Salz (Verbindung I-31)

200 mg (±)-1-(2,4-Dimethoxy-phenylsulfonyl)-3-(2-ethoxy-pyridin-3-yl)-3-{2-[4-(4-methylpiperazin-1-yl)-piperidin-1-yl]-2-oxo-ethyl}-2-oxo-2,3-dihydro-1H-indol-5-carbonitril, Trifluoressigsäure-Salz aus Beispiel 6 wurden über eine chirale präparative Säule (Chiralcell OD, Fluss 55 mL/min) mit n-Heptan / Ethanol (7 : 3) als Elutionsmittel getrennt. Man erhielt 13 mg des zuerst eluierenden Enantiomers mit positivem Drehwert (Beispiel 6A) und 9 mg des später eluierenden Enantiomers mit negativem Drehwert (Beispiel 6B).

### Beispiel 6A:

### (+)1-(2,4-Dimethoxy-phenylsulfonyl)-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril

### (Verbindung I-31A)

ESI-MS [M+H⁺] = 703
HPLC (Chiralcel OD 0.46 cm x 25 cm ; n-Heptan / Ethanol 7:3) R_{f} = 12.9 min
[α]₂₀^{D} = +13 (c 0.1, CHCl₃)
¹H-NMR ([d₆]-DMSO, 500 MHz) δ [ppm] = 8.10 (1H), 7.95 (2H), 7.85 (1H), 7.80 (1H), 7.55-7.60 (1H), 7.05 (1H), 6.65 (2H), 4.10 (2H), 3.80-4.05 (6H), 3.60 (3H), 3.15-3.30 (1H), 2.90 (1H), 2.25-2.50 (9H), 2.15 (3H), 1.70 (1H), 1.55 (1H), 1.20 (2H), 1.05 (3H), 0.80 (1H).

### Beispiel 6B:

### (-)1-(2,4-Dimethoxy-phenylsulfonyl)-3-(2-ethoxy-pyridin-3-yl)-3-{2-[4-(4-methyl-piperazin-1-yl)-piperidin-1-yl]-2-oxo-ethyl}-2-oxo-2,3-dihydro-1H-indol-5-carbonitril

### (Verbindung I-31B)

ESI-MS [M+H⁺] = 703
HPLC (Chiralcel OD 0.46 cm x 25 cm ; n-Heptan / Ethanol 7:3) R_{f} = 17.3 min
¹H-NMR (d₆]-DMSO, 500 MHz) δ [ppm] = 8.10 (1H), 7.95 (2H), 7.85 (1H), 7.80 (1H), 7.55-7.60 (1H), 7.05 (1H), 6.65 (2H), 4.10 (2H), 3.80-4.05 (6H), 3.60 (3H), 3.15-3.30 (1H), 2.90 (1H), 2.25-2.50 (9H), 2.15 (3H), 1.70 (1H), 1.55 (1H), 1.20 (2H), 1.05 (3H), 0.80 (1H).

### BEISPIEL 9A UND BEISPIEL 9B:

Racematspaltung von 1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril, Trifluoressigsäure-Salz (Verbindung I-1)

200 mg (±)-1-(2,4-Dimethoxy-phenylsulfonyl)-3-(2-ethoxy-pyridin-3-yl)-3-{2-[4-(1-methylpiperidin-4-yl)-piperazin-1-yl]-2-oxo-ethyl}-2-oxo-2,3-dihydro-1H-indol-5-carbonitril, Trifluoressigsäure-Salz aus Beispiel 9 wurden über eine chirale präparative Säule (Chiralcell OD, Fluss 55 mL/min) mit n-Heptan / Ethanol (7 : 3) als Elutionsmittel getrennt. 41 mg des zuerst eluierenden Enantiomers mit positivem Drehwert (Beispiel 9A) und 23 mg des später eluierenden Enantiomers mit negativem Drehwert (Beispiel 9B).

### Beispiel 9A:

### (+)1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril

### (Verbindung I-1A)

ESI-MS [M+H⁺] = 703
HPLC (Chiralcel OD 0.46 cm x 25 cm ; n-Heptan / Ethanol 7:3) R_{f} = 17.1 min
[α]₂₀^{D} = +9 (c 0.1, CHCl₃)
¹H-NMR ([d₆]-DMSO, 500 MHz) δ [ppm] = 8.10 (1H), 7.95 (2H), 7.85 (1H), 7.80 (1H), 7.60 (1H), 7.05 (1H), 6.65 (2H), 4.10 (2H), 3.90 (1H), 3.85 (3H), 3.60 (3H), 3.45 (2H), 3.25 (1H), 3.10 (1H), 3.00 (1H), 2.75 (2H), 2.40 (2H), 2.05-2.25 (6H), 1.80 (2H), 1.65 (2H), 1.40 (2H), 1.05 (3H).

### Beispiel 9B:

### (-)1-(2,4-Dimethoxy-phenylsulfonyl)-3-(2-ethoxy-pyridin-3-yl)-3-{2-[4-(1-methyl-piperidin-4-yl)-piperazin-1-yl]-2-oxo-ethyl}-2-oxo-2,3-dihydro-1H-indol-5-carbonitril (Verbindung I-1B)

ESI-MS [M+H⁺] = 703
HPLC (Chiralcel OD 0.46 cm x 25 cm ; n-Heptan / Ethanol 7:3) R_{f} = 26.3 min
[α]₂₀^{D} = -14 (c 0.1, CHCl₃)
¹H-NMR ([d₆]-DMSO, 500 MHz) δ [ppm] = 8.10 (1H), 7.95 (2H), 7.85 (1H), 7.80 (1H), 7.60 (1H), 7.05 (1H), 6.65 (2H), 4.10 (2H), 3.90 (1H), 3.85 (3H), 3.60 (3H), 3.45 (2H), 3.25 (1H), 3.10 (1H), 3.00 (1H), 2.75 (2H), 2.40 (2H), 2.05-2.25 (6H), 1.80 (2H), 1.65 (2H), 1.40 (2H), 1.05 (3H).

### Beispiel 10B:

### (-)1-(2,4-Dimethoxy-phenylsulfonyl)-3-(2-ethoxy-pyridin-3-yl)-3-{2-[4-(1-ethyl-piperidin-4-yl)-piperazin-1-yl]-2-oxo-ethyl}-2-oxo-2,3-dihydro-1H-indol-5-carbonitril (Verbindung I-7B)

Beispiel 10B wurde in Analogie zu Beispiel 9B unter Verwendung von 1-(1-Ethyl-piperidin-4-yl)-piperazin statt 1-(1-Methyl-piperidin-4-yl)-piperazin als Amin X hergestellt.
ESI-MS [M+H⁺] = 717

### Beispiel 11B

### 1-(2,4-Dimethoxy-phenylsulfonyl)-3-(2-ethoxy-pyridin-3-yl)-3-[2-(1'-methyl-[4,4']bipiperidinyl-1-yl)-2-oxo-ethyl]-2-oxo-2,3-dihydro-1H-indole-5-carbonitril (Verbindung I-61B)

Beispiel 11B kann in Analogie zu Beispiel 9B unter Verwendung von 1-Methyl-[4,4']bipiperidin statt 1-(1-Methyl-piperidin-4-yl)-piperazin als Amin X hergestellt werden.
ESI-MS [M+H⁺] = 702

### IV. BESTIMMUNG DER BIOLOGISCHEN AKTIVITÄT

### 1. Vasopressin V1b Rezeptorbindungstest:

### Substanzen:

Die Testsubstanzen wurden in einer Konzentration von 10⁻² M in DMSO gelöst und in DMSO auf 5x10⁻⁴ M bis 5x10⁻⁹ M weiter verdünnt. Diese DMSO-Vorverdünnungsreihe wurde mit Testpuffer 1:10 verdünnt. Im Testansatz wurde die Substanzkonzentration nochmals 1:5 verdünnt (2 % DMSO im Ansatz).

### Membran-Präparation:

CHO-K1 Zellen mit stabil exprimiertem humanem Vasopressin V1 b Rezeptor (Klon 3H2) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspuffer war: 50 mM Tris, 10 mM MgCl₂, 0.1 % BSA, pH 7.4.
Im Testansatz (250 µl) wurden Membranen (50 µg/ml Protein in Inkubationspuffer) von CHO-K1-Zellen mit stabil exprimierten humanen V1b Rezeptoren (Zelllinie hV1b_3H2_CHO) mit 1,5 nM ³H-AVP (8-Arg-Vasopressin, PerkinElmer #18479) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 M AVP (Bachem # H1780) bestimmt. Alle Bestimmungen wurden als Dreifachbestimmungen durchgeführt. Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt. Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten humanen V1b-Rezeptoren beträgt 0,4 nM und wurde zur Bestimmung des Ki-Wertes herangezogen.

### 2. Vasopressin V1a Rezeptorbindungstest:

### Substanzen:

Die Testsubstanzen wurden in einer Konzentration von 10⁻² M in DMSO gelöst. Die weitere Verdünnung dieser DMSO-Lösungen erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V1a Rezeptor (Klon 5) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspufferwar: 50 mM Tris, 10 mM MgCl₂, 0.1% BSA, pH 7.4.
Im Testansatz (250 µl) wurden Membranen (20 µg/ml Protein in Inkubationspuffer) von CHO-K1-Zellen mit stabil exprimierten humanen V1a Rezeptoren (Zelllinie hV1a_5_CHO) mit 0.04 nM ¹²⁵I-AVP (8-Arg-Vasopressin, NEX 128) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.
Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.
Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ¹²⁵I-AVP zu den rekombinanten hV1a-Rezeptoren wurde in Sättigungsexperimenten bestimmt. Ein Kd-Wert von 1,33 nM wurde zur Bestimmung des Ki-Wertes herangezogen.

### 3. Vasopressin V2 Rezeptorbindungstest:

### Substanzen:

Die Testsubstanzen wurden in einer Konzentration von 10⁻² M in DMSO gelöst. Die weitere Verdünnung dieser DMSO-Lösung erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V2 Rezeptor (Klon 23) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1 h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.
Inkubationspuffer war: 50 mM Tris, 10 mM MgCl₂, 0.1% BSA, pH 7.4.
Im Testansatz (250 µl) wurden Membranen (50 µg/ml Protein in Inkubationspuffer) von CHO-K1-Zellen mit stabil exprimierten humanen V2 Rezeptoren (Zelllinie hV2_23_CHO) mit 1-2 nM ³H-AVP (8-Arg-Vasopressin, PerkinElmer #18479) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.
Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.
Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten hV2-Rezeptoren beträgt 2,4 nM und wurde zur Bestimmung des Ki-Wertes herangezogen.

### 4. Oxytozin-Rezeptorbindungstest

### Substanzen:

Die Substanzen wurden in einer Konzentration von 10⁻² M in DMSO gelöst und mit Inkubationspuffer (50 mM Tris, 10 mM MgCl₂, 0.1% BSA, pH 7,4) verdünnt.

### Zellpräparation:

Konfluente HEK-293 Zellen mit transient exprimierenden rekombinanten humanen Oxytozinrezeptoren wurden bei 750 x g für 5 Minuten bei Raumtemperatur zentrifugiert. Der Rückstand wurde in eiskaltem Lysispuffer (50 mM Tris-HCl, 10 % Glycerin, pH 7,4 und Roche Complete Protease-Inhibitor) aufgenommen und 20 Minuten bei 4 °C einem osmotischen Schock unterworfen. Danach wurden die lysierten Zellen bei 750 x g für 20 Minuten bei 4°C zentrifugiert, der Rückstand in Inkubationspuffer aufgenommen und Aliquots von 10⁷ Zellen/ml hergestellt. Die Aliquots wurden bis zur Verwendung bei -80°C eingefroren.

### Bindungstest:

Am Tag des Versuches wurden die Zellen aufgetaut, mit Inkubationspuffer verdünnt und mit einer Multipette Combitip (Eppendorf, Hamburg) homogenisiert. Der Reaktionsansatz von 0,250 ml setzte sich zusammen aus 2 bis 5x10⁴ rekombinante Zellen, 3-4 nM ³H-Oxytozin (PerkinElmer, NET 858) in Anwesenheit von Testsubstanz (Hemmkurve) oder nur Inkubationspuffer (totale Bindung). Die unspezifische Bindung wurde mit 10⁻⁶ M Oxytozin (Bachem AG, H2510) bestimmt. Dreifachbestimmungen wurden angesetzt. Gebundener und freier Radioligand wurden getrennt durch Filtration unter Vakuum mit Whatman GF/B Glasfaserfilter mit Hilfe eines Skatron Cell Harvester 7000. Die gebundene Radioaktivität wurde durch Flüssigkeitszintillationsmessung in einem Tricarb Beta-Zählgerät, Modell 2000 oder 2200CA (Packard) bestimmt.

### Auswertung:

Die Bindungsparameter wurden durch nicht-lineare Regressionsanalyse berechnet (SAS), analog zum Programm LIGAND von Munson und Rodbard (Analytical Biochem 1980; 107: 220-239). Der Kd-Wert von ³H-Oxytozin zu den rekombinanten hOT-Rezeptoren beträgt 7.6 nM und wurde zur Bestimmung des Ki-Wertes herangezogen.

### 5. Bestimmung der mikrosomalen Halbwertszeit:

Die metabolische Stabilität der erfindungsgemäßen Verbindungen wurde in dem folgenden Test bestimmt.

Die Testsubstanzen werden in einer Konzentration von 0.5 µM wie folgt inkubiert:

In Mikrotiterplatten werden 0.5 µM Testsubstanz zusammen mit Lebermikrosomen verschiedener Spezies (von Ratte, Mensch oder andere Spezies) (0.25 mg mikrosomales Protein/ml) in 0.05M Kalium-Phosphatpuffer pH 7,4 bei 37 °C für 5 min vorinkubiert. Der Start der Reaktion erfolgt durch die Zugabe von NADPH (1 mg/mL). Nach 0, 5, 10, 15, 20 und 30 min werden 50 µl Aliquots entnommen und die Reaktion wird sofort mit dem gleichen Volumen an Acetonitril abgestoppt und runtergekühlt. Die Proben werden bis zur Analyse eingefroren. Mittels MSMS wird die verbliebene Konzentration an nicht abgebauter Testsubstanz bestimmt. Aus der Steigung der Kurve Signal Testsubstanz/Zeiteinheit wird die Halbwertszeit (T1/2) ermittelt, wobei die Halbwertszeit der Testsubstanz unter Annahme einer Kinetik erster Ordnung aus der zeitlichen Abnahme der Konzentration der Verbindung berechnet werden kann. Die mikrosomale Clearance (mCl) berechnet sich aus mCl= In2/T1/2 / (Gehalt an mikrosomalem Protein in mg/ml) x 1000 [ml/min/mg] (modifiziert nach Literaturstellen: Di, The Society for Biomoleculaur Screening, 2003, 453-462; Obach, DMD, 1999 vol 27. N 11, 1350-1359).

### 6. Methoden zur in vitro Bestimmung der Cytochrom P450 (CYP) Inhibierung

### Lumineszenzsubstrate für 2C9 und 3A4:

0.4 mg/ml humane Lebermikrosomen werden 10 min mit den zu untersuchenden Testsubstanzen (0-20 µM), den CYP-spezifischen Substraten, in 0.05 M Kaliumphosphatpuffer pH 7,4 bei 37 °C vorinkubiert. Das Cyp-spezifische Substrat für CYP 2C9 ist Luciferin H, für CYP 3A4 Luciferin BE. Die Reaktion wird durch Hinzufügen von NADPH gestartet. Nach 30 min Inkubation bei RT wird das Luciferin Detektionsreagenz hinzugefügt, und das entstandene Lumineszenzsignal gemessen (modifiziert nach Literaturstelle: Promega, Technical Bulletin P450-GLO^{™} Assays).

### Midazolam CYP 3A4 Time dependent Inhibition

Der Test besteht aus 2 Teilen. Einmal wird die Testsubstanz mit den Lebermikrosomen vorinkubiert (mit NADPH = Prä-Inkubation, danach Zugabe des Substrates, im 2 .Teil wird das Substrat und die Testsubstanz gleichzeitig zugegeben = Co-Inkubation.

### Prä-Inkubation:

0.05 mg/ml mikrosomales Protein (humane Lebermikrosomen) werden mit 0-10 µM (bzw 50 µM) Testsubstanz in 50 mM Kaliumphosphatpuffer 5 Min vorinkubiert. Die Reaktion wird mit NADPH gestartet. Nach 30 min werden 4 µM Midazolam (Endkonzentration) hinzugefügt und für weitere 10 Min inkubiert. 75 µl der Reaktionslösung werden nach 10 min entnommen und mit 150 µl Acetonitrillösung abgestoppt.

### Co-Inkubation:

0.05 mg/ml mikrosomales Protein (humane Lebermikrosomen) werden mit 4 µM Midazolam (Endkonzentration) und 0-10 µM (bzw 50µM) Testsubstanz in 50 mM Kaliumphosphatpuffer 5 min vorinkubiert. Die Reaktion wird mit NADPH gestartet. 75 µl der Reaktionslösung werden nach 10 min entnommen und mit 150 µl Acetonitrillösung abgestoppt. Die Proben werden bis zur MSMS-Analytik eingefroren (modifiziert nach Literaturstellen: Obdach, Journal of Pharmacology & Experimental Therapeutics, Vol 316, 1, 336-348, 2006; Walsky, Drug Metabolism and Disposition Vol 32, 6, 647-660, 2004).

### 7. Methode zur Bestimmung der Wasserlöslichkeit (in mg/ml)

Die Wasserlöslichkeit der erfindungsgemässen Verbindungen kann zum Beispiel nach der sogenannten "shake flask" Methode bestimmt werden (gemäss ASTM International: E 1148-02, Standard test methods for measurement of aqueous solubility, Book of Standards Volume 11.05*.).* Dabei wird ein Überschuss der festen Verbindung in eine Pufferlösung mit einem bestimmten pH-Wert (zum Beispiel Phosphatpuffer pH 7.4) gegeben und die entstehende Mischung geschüttelt oder gerührt, bis sich das Gleichgewicht eingestellt hat (typischerweise 24 oder 48 Stunden, manchmal auch bis zu 7 Tage). Anschließend wird der ungelöste Feststoff durch Filtrieren oder Zentrifugieren abgetrennt und die Konzentration der gelösten Verbindung durch UV-Spektroskopie oder HochdruckflüssigkeitsChromatographie (HPLC) anhand einer entsprechenden Kalibrierkurve bestimmt.

### 8. Ergebnisse

Die Ergebnisse der Rezeptor-Bindungsuntersuchungen sind als Rezeptorbindungskonstanten [Kᵢ(V1b), Kᵢ(V1a), Kᵢ(V2), Kᵢ(OT)] ausgedrückt. Die Ergebnisse der Untersuchung der metabolischen Stabilität sind als mikrosomale Clearance (mCl) angegeben.

In diesen Tests zeigen die erfindungsgemäßen Verbindungen sehr hohe Affinitäten für den V1b-Rezeptor (höchstens 300 nM, oder höchstens 30 nM, häufig < 3 nM). Außerdem zeigen die Verbindungen auch hohe Selektivitäten gegenüber dem V1a-Rezeptor. Zudem besitzen sie eine gute metabolische Stabilität, gemessen als mikrosomale Clearance.

Die Ergebnisse sind in Tabelle 2 aufgeführt. Die Nummern der Verbindungen beziehen sich auf die Synthesebeispiele.

**Tabelle 2**

| Beispiel | Kᵢ(h-V1b)* [nM] | Kᵢ(h-V1a)/Kᵢ(h-V1b)* | Humane mikrosomale Clearance [µl min⁻¹ mg⁻¹] |
|---|---|---|---|
| 1 | ++ | ++ | +++ |
| 2 | +++ | +++ | +++ |
| 3 | ++ | +++ | +++ |
| 4 | ++ | +++ | ++ |
| 5 | ++ | ++ | ++ |
| 6 | +++ | +++ | +++ |
| 7 | ++ | +++ | +++ |
| 8 | ++ | +++ | +++ |
| 9 | +++ | +++ | +++ |
| 10B | +++ | +++ | +++ |

| | | | |
|---|---|---|---|
| * h = human | | | |

### Schlüssel:

| | Kᵢ(V1b) | Kᵢ(h-V1a)/Kᵢ(h-V1b) | Humane mikrosomale Clearance |
|---|---|---|---|
| + | 300 - >30 nM | 10 - < 15 | > 200 - 300 µl min⁻¹ mg⁻¹ |
| ++ | 3-30nM | 15-30 | 100 - 200 µl min⁻¹ mg⁻¹ |
| +++ | <3nM | > 30 | < 100 µl min⁻¹ mg⁻¹ |

## Patentansprüche

1. Verbindungen der Formel I 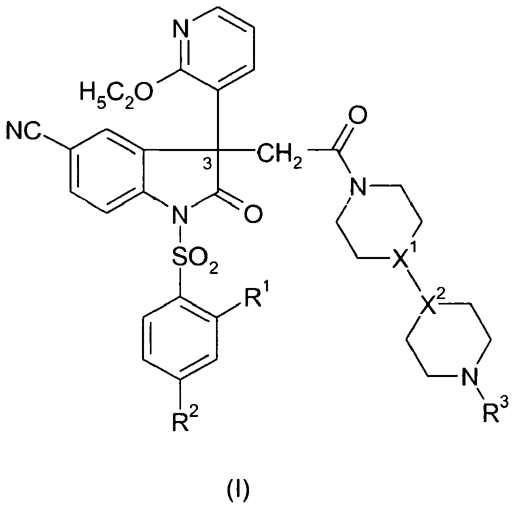 worin
R¹ für Wasserstoff, Methoxy oder Ethoxy steht;
R² für Wasserstoff oder Methoxy steht;
R³ für Wasserstoff, Methyl, Ethyl, n-Propyl oder Isopropyl steht;
X¹ und X² für N oder CH stehen, unter der Maßgabe, dass X¹ und X² nicht gleichzeitig für N stehen;
sowie deren pharmazeutisch verträglichen Salze.

2. Verbindungen nach Anspruch 1, worin R¹ für Wasserstoff oder Methoxy steht.

3. Verbindungen nach einem der vorhergehenden Ansprüche, worin R³ für Wasserstoff, Methyl oder Ethyl steht.

4. Verbindungen nach einem der vorhergehenden Ansprüche, worin X¹ für N steht und X² für CH.

5. Verbindungen nach einem der Ansprüche 1 bis 3, worin X¹ für CH steht und X² für N.

6. Verbindungen nach einem der vorhergehenden Ansprüche, worin
R¹ für Methoxy steht;
R² für Methoxy steht;
R³ für Methyl steht;
X¹ für CH steht; und
X² für N steht.

7. Verbindungen nach einem der Ansprüche 1 bis 5, worin
R¹ für Methoxy steht;
R² für Methoxy steht;
R³ für Methyl steht;
X¹ für N steht; und
X² für CH steht.

8. Verbindungen nach einem der Ansprüche 1 bis 5, worin
R¹ für Methoxy steht;
R² für Methoxy steht;
R³ für Methyl steht;
X¹ für CH steht; und
X² für CH steht.

9. Verbindungen nach einem der Ansprüche 1 bis 5, worin
R¹ für Methoxy steht;
R² für Methoxy steht;
R³ für Ethyl steht;
X¹ für CH steht; und
X² für N steht.

10. Verbindungen nach einem der Ansprüche 1 bis 5, worin
R¹ für Methoxy steht;
R² für Methoxy steht;
R³ für Ethyl steht;
X¹ für N steht; und
X² für CH steht.

11. Verbindungen nach einem der vorhergehenden Ansprüche, wobei es sich um das (-)-Enantiomer in einer Enantiomerenreinheit von wenigstens 90 % ee handelt.

12. Verbindung nach einem der Ansprüche 1 bis 10, wobei es sich um das Racemat handelt.

13. Pharmazeutisches Mittel, enthaltend wenigstens eine Verbindung der Formel I gemäß der Definition in einem der vorhergehenden Ansprüche und/oder wenigstens ein pharmazeutisch verträgliches Salz davon und wenigstens einen pharmazeutisch verträglichen Träger.

14. Verwendung von Verbindungen der Formel I gemäß der Definition in einem der Ansprüche 1 bis 12 oder von pharmazeutisch verträglichen Salzen davon zur Herstellung eines Medikaments zur Behandlung und/oder Prophylaxe von Vasopressin-abhängigen Krankheiten.

15. Verwendung nach Anspruch 14, zur Herstellung eines Medikaments zur Behandlung und/oder Prophylaxe von Krankheiten, die ausgewählt sind unter Diabetes, Insulin-Resistenz, Enuresis nocturna, Inkontinenz, Krankheiten, bei denen Blutgerinnungsstörungen auftreten,
Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, Reisekrankheit,
affektiven Störungen,
Angststörungen, stressabhängigen Angststörungen, Gedächtnisleistungsstörungen, Morbus Alzheimer,
Psychosen, psychotischen Störungen,
Cushing-Syndrom, sonstigen stress-abhängigen Krankheiten, Schlafstörungen,
depressiven Erkrankungen, vorzugsweise childhood onset mood disorders, vasomotorischen Symptomen, thermoregulatorischen Fehlfunktionen, Drogen-, Arzneimittel- und/oder durch sonstige Faktoren vermittelten Abhängigkeiten; von Stress bedingt durch den Entzug von einem oder mehreren die Abhängigkeit vermittelnden Faktoren; und/oder von Stress-induzierten Rückfällen in die Drogen-, Arzneimittel- und/oder durch sonstige Faktoren vermittelte Abhängigkeiten,
Schizophrenie, und Psychose,
und/oder zur Verzögerung der Miktion.

## Claims

1. Compounds of the formula I 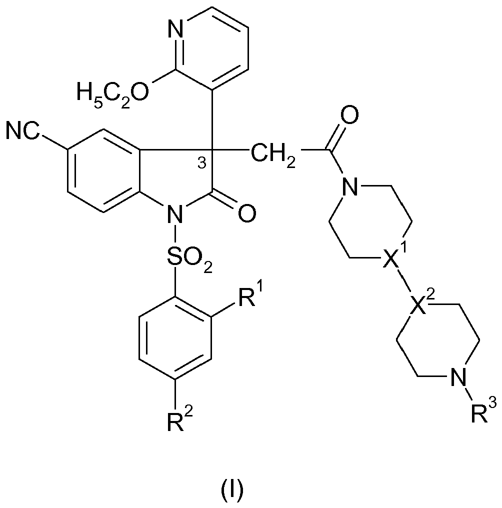 in which
R¹ is hydrogen, methoxy or ethoxy;
R² is hydrogen or methoxy;
R³ is hydrogen, methyl, ethyl, n-propyl or isopropyl;
X¹ and X² are N or CH, with the proviso that X¹ and X² are not simultaneously N;
and their pharmaceutically acceptable salts.

2. Compounds according to Claim 1, in which R¹ is hydrogen or methoxy.

3. Compounds according to any of the preceding claims, in which R³ is hydrogen, methyl or ethyl.

4. Compounds according to any of the preceding claims, in which X¹ is N and X² is CH.

5. Compounds according to any of Claims 1 to 3, in which X¹ is CH and X² is N.

6. Compounds according to any of the preceding claims, in which
R¹ is methoxy;
R² is methoxy;
R³ is methyl;
X¹ is CH; and
X² is N.

7. Compounds according to any of Claims 1 to 5, in which
R¹ is methoxy;
R² is methoxy;
R³ is methyl;
X¹ is N; and
X² is CH.

8. Compounds according to any of Claims 1 to 5, in which
R¹ is methoxy;
R² is methoxy;
R³ is methyl;
X¹ is CH; and
X² is CH.

9. Compounds according to any of Claims 1 to 5, in which
R¹ is methoxy;
R² is methoxy;
R³ is ethyl;
X¹ is CH; and
X² is N.

10. Compounds according to any of Claims 1 to 5, in which
R¹ is methoxy;
R² is methoxy;
R³ is ethyl;
X¹ is N; and
X² is CH.

11. Compounds according to any of the preceding claims, which is the (-) enantiomer which has an enantiomeric purity of at least 90% ee.

12. Compound according to any of Claims 1 to 10, which is the racemate.

13. Pharmaceutical composition comprising at least one compound of the formula I as defined in any of the preceding claims and/or at least one pharmaceutically acceptable salt thereof and at least one pharmaceutically acceptable carrier.

14. Use of compounds of the formula I as defined in any of Claims 1 to 12 or of pharmaceutically acceptable salts thereof for the manufacture of a medicament for the treatment and/or prophylaxis of vasopressin-dependent diseases.

15. Use according to Claim 14, for the manufacture of a medicament for the treatment and/or prophylaxis of diseases selected from diabetes, insulin resistance, nocturnal enuresis, incontinence, diseases in which impairments of blood clotting occur, hypertension, pulmonary hypertension, heart failure, myocardial infarction, coronary spasm, unstable angina, PTCA (percutaneous transluminal coronary angioplasty), ischemias of the heart, impairments of the renal system, edemas, renal vasospasm, necrosis of the renal cortex, hyponatremia, hypokalemia, Schwartz-Bartter syndrome, impairments of the gastrointestinal tract, gastritic vasospasm, hepatocirrhosis, gastric and intestinal ulcers, emesis, emesis occurring during chemotherapy, travel sickness, affective disorders, anxiety disorders, stress-dependent anxiety disorders, memory impairments, Alzheimer's disease, psychoses, psychotic disorders, Cushing's syndrome, other stress-dependent diseases, sleep disorders, depressive disorders, preferably, childhood onset mood disorders, vasomotor symptoms, thermoregulatory dysfunctions, drug or pharmaceutical dependencies and/or dependencies mediated by other factors; of stress caused by withdrawal of one or more factors mediating the dependence; and/or of stress-induced relapses into drug or pharmaceutical dependencies and/or dependencies mediated by other factors, schizophrenia, and psychosis, and/or for delaying micturition.

## Revendications

1. Composés de formule I 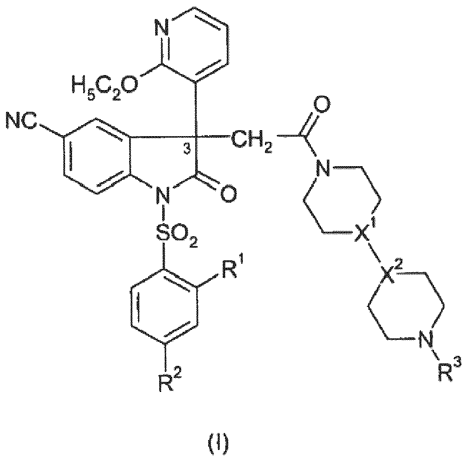 dans laquelle
R¹ représente un atome d'hydrogène, le groupe méthoxy ou éthoxy ;
R² représente un atome d'hydrogène ou le groupe méthoxy ;
R³ représente un atome d'hydrogène, le groupe méthyle, éthyle, n-propyle ou isopropyle ;
X¹ et X² représentent N ou CH, étant entendu que X¹ et X² ne représentent pas simultanément N ;
ainsi que leurs sels pharmaceutiquement acceptables.

2. Composés selon la revendication 1, dans lesquels R¹ représente un atome d'hydrogène ou le groupe méthoxy.

3. Composés selon l'une quelconque des revendications précédentes, dans lesquels R³ représente un atome d'hydrogène, le groupe méthyle ou éthyle.

4. Composés selon l'une quelconque des revendications précédentes, dans lesquels X¹ représente N et X² représente CH.

5. Composés selon l'une quelconque des revendications 1 à 3, dans lesquels X¹ représente CH et X² représente N.

6. Composés selon l'une quelconque des revendications précédentes, dans lesquels
R¹ représente le groupe méthoxy ;
R² représente le groupe méthoxy ;
R³ représente le groupe méthyle ;
X¹ représente CH ; et
X² représente N.

7. Composés selon l'une quelconque des revendications 1 à 5, dans lesquels
R¹ représente le groupe méthoxy ;
R² représente le groupe méthoxy ;
R³ représente le groupe méthyle ;
X¹ représente N ; et
X² représente CH.

8. Composés selon l'une quelconque des revendications 1 à 5, dans lesquels
R¹ représente le groupe méthoxy ;
R² représente le groupe méthoxy ;
R³ représente le groupe méthyle ;
X¹ représente CH ; et
X² représente CH.

9. Composés selon l'une quelconque des revendications 1 à 5, dans lesquels
R¹ représente le groupe méthoxy ;
R² représente le groupe méthoxy ;
R³ représente le groupe éthyle ;
X¹ représente CH ; et
X² représente N.

10. Composés selon l'une quelconque des revendications 1 à 5, dans lesquels
R¹ représente le groupe méthoxy ;
R² représente le groupe méthoxy ;
R³ représente le groupe éthyle ;
X¹ représente N ; et
X² représente CH.

11. Composés selon l'une quelconque des revendications précédentes, qui consistent en l'énantiomère (-) en une pureté d'énantiomère d'au moins 90 % ee.

12. Composé selon l'une quelconque des revendications 1 à 10, qui consiste en le racémate.

13. Produit pharmaceutique, contenant au moins un composé de formule I selon la définition dans l'une quelconque des revendications précédentes et/ou au moins un sel pharmaceutiquement acceptable d'un tel composé et au moins un véhicule pharmaceutiquement acceptable.

14. Utilisation des composés de formule I selon la définition dans l'une quelconque des revendications 1 à 12 ou de sels pharmaceutiquement acceptables de tels composés, pour la fabrication d'un médicament destiné au traitement et/ou à la prophylaxie de maladies dépendant de la vasopressine.

15. Utilisation selon la revendication 14, pour la fabrication d'un médicament destiné au traitement et/ou à la prophylaxie de maladies qui sont choisies parmi le diabète, la résistance à l'insuline, l'énurésie nocturne, l'incontinence, les maladies dans lesquelles apparaissent des troubles de la coagulation du sang, l'hypertonie, l'hypertonie pulmonaire, l'insuffisance cardiaque, l'infarctus du myocarde, le spasme coronarien, l'angine instable, la PTCA (percutaneus transluminal coronary angioplasty), les ischémies du cour, les troubles du système rénal, les oedèmes, le vasospasme rénal, la nécrose du cortex rénal, l'hyponatrémie, l'hypokaliémie, le syndrome de Schwartz-Bartter, les troubles du tractus gastrointestinal, le vasospasme gastrique, l'hépatocirrhose, l'ulcère gastrique et intestinal, les vomissements, les vomissements apparaissant lors de la chimiothérapie, la maladie des transports,
les troubles affectifs,
les troubles anxieux, les troubles anxieux dépendant du stress,
les troubles de la fixation mnémonique, la maladie d'Alzheimer,
les psychoses, les troubles psychotiques,
le syndrome de Cushing, d'autres maladies dépendant du stress,
les troubles du sommeil,
les maladies dépressives, de préférence childhood onset mood disorders,
les symptômes vasomoteurs, les dysfonctionnements thermorégulateurs,
les dépendances provoquées par des drogues, des médicaments et/ou par d'autres facteurs ; du stress causé par le retrait d'un ou plusieurs facteurs provoquant la dépendance ; et/ou de rechutes induites par le stress dans les dépendances provoquées par des drogues, des médicaments et/ou par d'autres facteurs,
la schizophrénie, et la psychose,
et/ou au retardement de la miction.
